# EUROPEAN PATENT SPECIFICATION

(11) **EP 0 551 324 B1**
(45) Date of publication and mention of the grant of the patent: **09.07.1997**
(21) Application number: 91917117.3
(22) Date of filing: 01.10.1991
(51) Int. Cl.: C12N 15/31, C12N 15/62, C12Q 1/68, G01N 33/569, C07H 21/04

(54) **SALMONELLA POLYNUCLEOTIDE SEQUENCE**
POLYNUKLEOTIDSEQUENZ VON SALMONELLA
SEQUENCE DE POLYNUCLEOTIDES DE LA SALMONELLE

(30) Priority: 01.10.1990 GB 9021338; 17.10.1990 GB 9022570
(43) Date of publication of application: 21.07.1993
(73) Proprietor: THE MINISTER OF AGRICULTURE FISHERIES AND FOOD IN HER BRITANNIC MAJESTY'S GOVERNMENT OF THE UNITED KINGDOM OF GREAT BRITAIN, London SW1A 2HH (GB)
(72) Inventor: WOODWARD, Martin, John 23 Burnsall Close, Hampshire GU14 8NN (GB)
(74) Representative: Skelton, Stephen Richard
(86) International application number: GB9101691
(87) International publication number: WO9206198

(56) References cited:
- EP-A- 0 383 509
- WO-A-89/10967
- JOURNAL OF BACTERIOLOGY, vol. 170, no. 9, September 1988, AMERICAN SOCIETY FOR MICROBIOLOGY, pages 4216-4222; FEUTRIER, J. ET AL
- JOURNAL OF BACTERIOLOGY, vol. 168, no. 1, October 1986, AMERICAN SOCIETY FOR MICROBIOLOGY, pages 221-227; FEUTRIER, J. ET AL
- JOURNAL OF GENERAL MICROBIOLOGY, vol. 136, no. 2, February 1990, COLCHESTER, GB, pages 265-272; RADFORD, A.J. ET AL

## Description

This invention relates to polynucleotides (DNA) comprising a sequence characteristic of certain serotypes of the genus Salmonella; to the use of sequences comprising the characteristic sequence as polymerase chain reaction and hybridization targets for the identification of said serotypes and to test kits for this; to the use of polynucleotides comprising the sequence to transform suitable host cells to make them capable of expressing amino acid sequences characteristic of said strains; to said amino acid sequence when so expressed and kits containing them; and to plasmids and transformed cells containing said polynucleotide sequences.

Organisms of the genus Salmonella, in particular S. enteritidis, S. dublin and S. typhimurium are responsible for infective food poisoning caused by their ingestion in contaminated food. Infection with Salmonella may also occur as a result of contact with contaminated materials. Once ingested, Salmonella is able to establish itself in the gut and multiply rapidly, resulting in the appearance of clinical symptoms several days after the initial ingestion.

It is therefore highly desirable to provide test methods by means of which Salmonella organisms may be detected. In recent years immunological tests have been devised in which specific antibodies, particularly monoclonal antibodies ("MABs"), to specific antigens are raised and which by exploitation of the antigen - antibody specific binding reaction the presence of the antigen can be detected. Such tests are fast and very specific.

It is known that Salmonella organisms have fimbria like structures on their surface (Duguid; J. P and R. R. Gillies (1958) J. Pathol. Bacteriol. 75:519-520) and published evidence (Clegg, S., and G. F. Gerlach (1987) J. Bacteriol. 169:934-938.) suggests that there are antigenically distinct types of fimbriae, ie. possessing specific epitopes on the fimbrial antigens. The possibility of immunogenic tests for Salmonella, at least S. enteritidis, based upon these fimbrial antigens has been suggested (MAFF, Central Veterinary Laboratory "Animal Health" (1989):33). Methods of raising MABs to antigens on the surface of micro-organisms such as Salmonella are generally known.

Unfortunately known methods for raising antibodies to Salmonella surface antigens only go part way toward providing an immunological test for Salmonella. The basis of all these tests is to isolate micro-organisms from a sample suspected of harbouring Salmonella, then to grow the micro-organisms in vitro in a suitable culture medium until a quantity of the Salmonella sufficient to detect by such a test is believed to be present in the medium, and then applying the test. A problem occurs in that although Salmonella micro-organisms produce their fimbrial antigen when they grow in vivo, eg. in the gut, in animal tissues or fluids, in food products and in some natural environments, many of the fimbrial antigens are not produced when they are grown in vitro.

The present inventors have determined the polynucleotide sequence responsible for producing a characteristic fimbrial antigen, Salmonella enteritidis fimbrial antigen (SEFA). SEFA has an amino acid sequence forming an epitope on the fimbria 'in vivo' which is specifically found encoded by the DNA of the species S. enteritidis and some strains of the species S. dublin and S. Moscow but which is apparently absent in virtually all other serotypes. The identification and recognition of the significance of this sequence provides the basis for a number of determinative tests for the presence of the particular organisms or DNA/RNA derived from them and provides a method for production of transformed organisms capable of expressing SEFA or epitopic parts of SEFA.

The amino acid sequence of SEFA is provided below; it is of course to be expected that allelic variation will occur in some organisms.

### AMINO ACID SEQUENCE OF SALMONELLA ENTERITIDIS FIMBRIAL ANTIGEN

M L I V D F W R F C N M R K S A S A V A V L A L I A C G S A H A A G F
V G N K A E V Q A A V T I A A Q N T T S A N W S Q D P G F T G P A V A
A G Q K V G T L S I T A T G P H N S V S I A G K G A S V S G G V A T V
P F V D G Q G Q P V F R G R I Q G A N I N D Q A N T G I D G L A G W R
V A S S Q E T L N V P V T T F G K S T L P A G T F T A T F Y V Q Q Y Q
N

The codes above are standard codes, read amino-terminal to carboxy -terminal, left to right, M to N, according to the following key:

| Amino acid | | | | | |
|---|---|---|---|---|---|
| Alanine | A | Lysine | K | Arginine | R |
| Methionine | M | Asparagine | N | Phenylalanine | F |
| Aspartic acid | D | Proline | P | Cysteine | C |
| Pyroglutamyl | *E | Glutamic acid | E | Serine | S |
| Glutamine | Q | Threonine | T | Glycine | G |
| Tryptophan | W | Histidine | H | Tyrosine | Y |
| Isoleucine | I | Valine | V | Leucine | L |

Thus in its broadest form the present invention relates to DNA which encodes SEFA, or an epitopic part thereof or an allelic variant of either, each being characterised in that they bind to the SEFA specific antibodies described below.

A first preferred aspect of the present invention provides recombinant DNA comprising the sequences I and II: Sequence I 
5'- G CTCAGAATAC AACATCAGCC AACTGGAGTC AGGAT -3'
3'- C GAGTCTTATG TTGTAGTCGG TTGACCTCAG TCCTA -5'
230 240 250
 
Sequence II

5'- CCTGG CTTTACAGGG CCTGCTGTTG CTGCTGGTCA GAAAGTTGGT
3'- GGACC GAAATGTCCC GGACGACAAC GACGACCAGT CTTTCAACCA
260 270 280 290 300

ACTCTCAGCA TTACTGCTAC TGGTCCACAT AACTCAGTAT CTATTGCAGG TAAAGGGGCT
TGAGAGTCGT AATGACGATG ACCAGGTGTA TTGAGTCATA GATAACGTCC ATTTCCCCGA
310 320 330 340 350 360

TCGGTATCTG GTGGTGTAGC CACTGTCCCG TTCGTTGATG GACAAGGACA GCCTGTTTT -3'
AGCCATAGAC CACCACATCG GTGACAGGGC AAGCAACTAC CTGTTCCTGT CGGACAAAA -5'
370 380 390 400 410
 or sequences degenerately equivalent thereto.

The numerals below each ten base pair sequence in sequence I and II above are those designating the position of the individual base pairs in a larger characteristic sequence that comprises the entire SEFA antigen coding polynucleotide sequence.

By 'degenerately equivalent' is meant that substitute codons are present, these being codons which though they differ in their nucleotide base sequence from the codons identified in sequences I and II above, still code for the same amino acid, as will be understood by a man skilled in the art.

Preferred recombinant DNA of the invention, comprising sequences I and II, is that comprising sequences III and IV: 
Sequence III

5'- ATGCTAAT AGTTGATTTT TGGAGATTTT GTAATATGCG TAAATCAGCA
3'- TACGATTA TCAACTAAAA ACCTCTAAAA CATTATACGC ATTTAGTCGT
80 90 100 110 120

TCTGCAGTAG CAGTTCTTGC TTTAATTGCA TGTGGCAGTG CCCACGCAGC TGGCTTTGTT
AGACGTCATC GTCAAGAACG AAATTAACGT ACACCGTCAC GGGTGCGTCG ACCGAAACAA
130 140 150 160 170 180

GGTAACAAAG CAGAGGTTCA GGCAGCGGTT ACTATTGCAG CTCAGAATAC AACATCAGCC
CCATTGTTTC GTCTCCAAGT CCGTCGCCAA TGATAACGTC GAGTCTTATG TTGTAGTCGG
190 200 210 220 230 240

AACTGGAGTC AGGAT -3'
TTGACCTCAG TCCTA -5'
250
 
Sequence IV

5'- CCTGG CTTTACAGGG CCTGCTGTTG CTGCTGGTCA GAAAGTTGGT
3'- GGACC GAAATGTCCC GGACGACAAC GACGACCAGT CTTTCAACCA
260 270 280 290 300

ACTCTCAGCA TTACTGCTAC TGGTCCACAT AACTCAGTAT CTATTGCAGG TAAAGGGGCT
TGAGAGTCGT AATGACGATG ACCAGGTGTA TTGAGTCATA GATAACGTCC ATTTCCCCGA
310 320 330 340 350 360

TCGGTATCTG GTGGTGTAGC CACTGTCCCG TTCGTTGATG GACAAGGACA GCCTGTTTTC
AGCCATAGAC CACCACATCG GTGACAGGGC AAGCAACTAC CTGTTCCTGT CGGACAAAAG
370 380 390 400 410 420
 
CGTGGGCGTA TTCAGGGAGC CAATATTAAT GACCAAGCAA ATACTGGAAT TGACGGGCTT
GCACCCGCAT AAGTCCCTCG GTTATAATTA CTGGTTCGTT TATGACCTTA ACTGCCCGAA
430 440 450 460 470 480

GCAGGTTGGC GAGTTGCCAG CTCTCAAGAA ACGCTAAATG TCCCTGTCAC AACCTTTGGT
CGTCCAACCG CTCAACGGTC GAGAGTTCTT TGCGATTTAC AGGGACAGTG TTGGAAACCA
490 500 510 520 530 540

AAATCGACCC TGCCAGCAGG TACTTTCACT GCGACCTTCT ACGTTCAGCA GTATCAAAAC -3'
TTTAGCTGGG ACGGTCGTCC ATGAAAGTGA CGCTGGAAGA TGCAAGTCGT CATAGTTTTG -5'
550 560 570 580 590 600
 or sequences degenerately equivalent thereto.

The significance of sequences III and IV is that when they run contiguously together, such that the -3' end of the top strand of sequence III is immediately followed by the top strand 5'- end of sequence IV, they consist of the polynucleotide sequence that encodes the amino acid sequence for SEFA (said upper strand).

Thus polynucleotide sequence encoding SEFA is on the upper strand as shown above beginning ATGCTAATAG on III and ending GTATCAAAAC on IV. Further sequences which comprise suitable flanking sequences for control of amino acid sequence expression may be produced by genetic engineering techniques from this continuous sequence.

The invention further provides recombinant DNA comprising sequence III and IV, in the form of that comprising sequences V and VI: 
Sequence V

5'- GATCCTTGTT TTTTTTCTTA AATTTTTAAA ATGGCGTGAG TATATTAGCA TCCGCACAGA
3'- CTAGGAACAA AAAAAAGAAT TTAAAAATTT TACCGCACTC ATATAATCGT AGGCGTGTCT
10 20 30 40 50 60

TAAATTGTGC GAATGCTAAT AGTTGATTTT TGGAGATTTT GTAATATGCG TAAATCAGCA
ATTTAACACG CTTACGATTA TCAACTAAAA ACCTCTAAAA CATTATACGC ATTTAGTCGT
70 80 90 100 110 120

TCTGCAGTAG CAGTTCTTGC TTTAATTGCA TGTGGCAGTG CCCACGCAGC TGGCTTTGTT
AGACGTCATC GTCAAGAACG AAATTAACGT ACACCGTCAC GGGTGCGTCG ACCGAAACAA
130 140 150 160 170 180

GGTAACAAAG CAGAGGTTCA GGCAGCGGTT ACTATTGCAG CTCAGAATAC AACATCAGCC
CCATTGTTTC GTCTCCAAGT CCGTCGCCAA TGATAACGTC GAGTCTTATG TTGTAGTCGG
190 200 210 220 230 240

AACTGGAGTC AGGAT -3'
TTGACCTCAG TCCTA -5'
250
 
Sequence VI

5'- CCTGG CTTTACAGGG CCTGCTGTTG CTGCTGGTCA GAAAGTTGGT
3'- GGACC GAAATGTCCC GGACGACAAC GACGACCAGT CTTTCAACCA
260 270 280 290 300

ACTCTCAGCA TTACTGCTAC TGGTCCACAT AACTCAGTAT CTATTGCAGG TAAAGGGGCT
TGAGAGTCGT AATGACGATG ACCAGGTGTA TTGAGTCATA GATAACGTCC ATTTCCCCGA
310 320 330 340 350 360

TCGGTATCTG GTGGTGTAGC CACTGTCCCG TTCGTTGATG GACAAGGACA GCCTGTTTTC
AGCCATAGAC CACCACATCG GTGACAGGGC AAGCAACTAC CTGTTCCTGT CGGACAAAAG
370 380 390 400 410 420

CGTGGGCGTA TTCAGGGAGC CAATATTAAT GACCAAGCAA ATACTGGAAT TGACGGGCTT
GCACCCGCAT AAGTCCCTCG GTTATAATTA CTGGTTCGTT TATGACCTTA ACTGCCCGAA
430 440 450 460 470 480

GCAGGTTGGC GAGTTGCCAG CTCTCAAGAA ACGCTAAATG TCCCTGTCAC AACCTTTGGT
CGTCCAACCG CTCAACGGTC GAGAGTTCTT TGCGATTTAC AGGGACAGTG TTGGAAACCA
490 500 510 520 530 540

AAATCGACCC TGCCAGCAGG TACTTTCACT GCGACCTTCT ACGTTCAGCA GTATCAAAAC
TTTAGCTGGG ACGGTCGTCC ATGAAAGTGA CGCTGGAAGA TGCAAGTCGT CATAGTTTTG
550 560 570 580 590 600

TAATTTAATT TAAACTTTAT AAATGCCCTC AATATGAGCG AGTTTGGATA ATTTTATTAT
ATTAAATTAA ATTTGAAATA TTTACGGGAG TTATACTCGC TCAAACCTAT TAAAATAATA
610 620 630 640 650 660
 
TTTAAAAATA TCTATTTTGA ATAGATAGGT TTTATGCTTC CATGCAAAAA CTTAAAGAGG
AAATTTTTAT AGATAAAACT TATCTATCCA AAATACGAAG GTACGTTTTT GAATTTCTCC
670 680 690 700 710 720

GATTATGTAT ATTTTGAATA AATTTATACG TAGAACTGTT ATCTTTTTCC TTTTTTTTGC
CTAATACATA TAAAACTTAT TTAAATATGC ATCTTGACAA TAGAAAAAGG AAAAAAAACG
730 740 750 760 770 780

TACCTTCCAA TTGCTTCTTC GGAAAGTAAA AAAATTGAGC AACCATTATT AACACAAAAA
ATGGAAGGTT AACGAAGAAG CCTTTCATTT TTTTAACTCG TTGGTAATAA TTGTGTTTTT
790 800 810 820 830 840

TATTATGGCC TAAGATTGGG CACTACACGT GTTATTTATA AAGAAGATGC TCCATCAACA
ATAATACCGG ATTCTAACCC GTGATGTGCA CAATAAATAT TTCTTCTACG AGGTAGTTGT
850 860 870 880 890 900

AGTTTTTGGA TTATGAATGA AAAAGAATAT CCAATCCTTG TTCAAACTCA AGTATATAAT
TCAAAAACCT AATACTTACT TTTTCTTATA GGTTAGGAAC AAGTTTGAGT TCATATATTA
910 920 930 940 950 960

GATGATAAAT CATCAAAAGC TCCATTTATT GTAACACCAC CTATTTTGAA AGTTGAAAGT
CTACTATTTA GTAGTTTTCG AGGTAAATAA CATTGTGGTG GATAAAACTT TCAACTTTCA
970 980 990 1000 1010 1020

AATGCGCGAA CAAGATTGAA GGTAATACCA ACAAGTAATC TATTCAATAA AAATGAGGAG
TTACGCGCTT GTTCTAACTT CCATTATGGT TGTTCATTAG ATAAGTTATT TTTACTCCTC
1030 1040 1050 1060 1070 1080
 
TCTTTGTATT GGTTGTGTGT AAAAGGAGTC CCACCACTAA ATGATAATGA AAGCAATAAT
AGAAACATAA CCAACACACA TTTTCCTCAG GGTGGTGATT TACTATTACT TTCGTTATTA
1090 1100 1110 1120 1130 1140

AAAAACAACA TAACTACGAA TCTTAATGTG AATGTGGTTA CGAATAGTTG TATTAAATTA
TTTTTGTTGT ATTGATGCTT AGAATTACAC TTACACCAAT GCTTATCAAC ATAATTTAAT
1150 1160 1170 1180 1190 1200

ATTTATAGGC CTAAAACTAT AGACTTAACG ACAATGGAGA TTGCAGATAA ATTAAAGTTA
TAAATATCCG GATTTTGATA TCTGAATTGC TGTTACCTCT AACGTCTATT TAATTTCAAT
1210 1220 1230 1240 1250 1260

GAGAGAAAAG GAAATAGTAT AGTTATAAAG AATCCAACAT CATCATATGT GAATATTGCA
CTCTCTTTTC CTTTATCATA TCAATATTTC TTAGGTTGTA GTAGTATACA CTTATAACGT
1270 1280 1290 1300 1310 1320

AATATTAAAT CTGGTAATTT AAGTTTTAAT ATTCCAAATG GATATATTGA GCCATTTGGA
TTATAATTTA GACCATTAAA TTCAAAATTA TAAGGTTTAC CTATATAACT CGGTAAACCT
1330 1340 1350 1360 1370 1380

TATGCTCAAT TACCTGGTGG AGTACATAGT AAAATAACTT TGACTATTTT GGATGATAAC
ATACGAGTTA ATGGACCACC TCATGTATCA TTTTATTGAA ACTGATAAAA CCTACTATTG
1390 1400 1410 1420 1430 1440

GGCGCTGAAA TTATAAGAGA ATTATTAGTT TAAGGTGTAA AACAAATGAA GAAAACCACA
CCGCGACTTT AATATTCTCT TAATAATCAA ATTCCACATT TTGTTTACTT CTTTTGGTGT
1450 1460 1470 1480 1490 1500
 
ATTACTCTAT TTGTTTTAAC CAGTGTATTT CACTCTGGAA ATGTTTTCTC CAGACAATAT
TAATGAGATA AACAAAATTG GTCACATAAA GTGAGACCTT TACAAAAGAG GTCTGTTATA
1510 1520 1530 1540 1550 1560

AATTTCGACT ATGGAAGTTT GAGTCTTCTC CCGGTGAGAA TGCATCTTTT CTAAGTGTTG
TTAAAGCTGA TACCTTCAAA CTCAGAAGAG GGCCACTCTT ACGTAGAAAA GATTCACAAC
1570 1580 1590 1600 1610 1620

AAACGCTTCC CTGGTAATTA TGTTGTTGAT GTATATTTGA ATAATCAGTT AAAAGAAACT
TTTGCGAAGG GACCATTAAT ACAACAACTA CATATAAACT TATTAGTCAA TTTTCTTTGA
1630 1640 1650 1660 1670 1680

ACTGAGTTGT ATTTCAAATC AATGACTCAG ACTCTAGAAC CATGCTTAAC AAAAGAAAAA
TGACTCAACA TAAAGTTTAG TTACTGAGTC TGAGATCTTG GTACGAATTG TTTTCTTTTT
1690 1700 1710 1720 1730 1740

CTTATAAAGT ATGGGATCGC CATCCAGGAG CTTCATGGGT TGCAGTTTGA TAATGAACAA
GAATATTTCA TACCCTAGCG GTAGGTCCTC GAAGTACCCA ACGTCAAACT ATTACTTGTT
1750 1760 1770 1780 1790 1800

TGCGTTCTCT TAGAGCATTC TCCTCTTTAA ATATACTTAT AACGCGGCTA ACCAAAGTTT
ACGCAAGAGA ATCTCGTAAG AGGAGAAATT TATATGAATA TTGCGCCGAT TGGTTTCAAA
1810 1820 1830 1840 1850 1860

GCTTTTAAAT GCACCATCTA AAATTCTATC TCCAATAGAC AGTGAAATTG CTGATGAAAA
CGAAAATTTA CGTGGTAGAT TTTAAGATAG AGGTTATCTG TCACTTTAAC GACTACTTTT
1870 1880 1890 1900 1910 1920
 
TATCTGGGAT GATGGCATTA ACGCTTTTCT TTTAAATTAC AGAGCTTAAT TATTTGCATT
ATAGACCCTA CTACCGTAAT TGCGAAAAGA AAATTTAATG TCTCGAATTA ATAAACGTAA
1930 1940 1950 1960 1970 1980

CTAAGGTTGG AGGAGAGAGA TTCATACTTT GGTCAAATTC AACCTTGGTT TTAATTTTGG
GATTCCAACC TCCTCTCTCT AAGTATGAAA CCAGTTTAAG TTGGAACCAA AATTAAAACC
1990 2000 2010 2020 2030 2040

TCCCTGGCGG CTAAGGAATC TATCATCTTG GCAAAACTTG TCAAGCGAAA AAAAATTTGA
AGGGACCGCC GATTCCTTAG ATAGTAGAAC AGTTTTGAAC AGTTCGCTTT TTTTTAAACT
2050 2060 2070 2080 2090 2100

ATCAGCATAT ATTTATGCTG AGCGAGGTTT AAAAAAAATA AAGAGCAAAC TAACAGTTGG
TAGTCGTATA TAAATACGAC TCGCTCCAAA TTTTTTTTAT TTCTCGTTTG ATTGTCAACC
2110 2120 2130 2140 2150 2160

GGACAAATAT ACCAGTGCAG ATTTATTCGA TAGCGTACCA TTTAGAGGCT TTTCTTTAAA
CCTGTTTATA TGGTCACGTC TAAATAAGCT ATCGCATGGT AAATCTCCGA AAAGAAATTT
2170 2180 2190 2200 2210 2220

TAAAGATGAA AGTATGATAC CTTTCTCACA GAGAACATAT TATCCAACAA TACGTGGTAT
ATTTCTACTT TCATACTATG GAAAGAGTGT CTCTTGTATA ATAGGTTGTT ATGCACCATA
2230 2240 2250 2260 2270 2280

TGCGAAAACC AATGCGACTG TAGAAGTAAG ACAAAATGGA TACTTGATAT ATTCTACTTC
ACGCTTTTGG TTACGCTGAC ATCTTCATTC TGTTTTACCT ATGAACTATA TAAGATGAAG
2290 2300 2310 2320 2330 2340

AGTCCCCCCC GGGCAATTCG AGATAGGTAG AGAACAAATT GCTGATC -3'
TCAGGGGGGG CCCGTTAAGC TCTATCCATC TCTTGTTTAA CGACTAG -5'
2350 2360 2370 2380
 or sequences degeneratively equivalent thereto.

For the purposes of expressing SEFA polypeptide or epitopic parts thereof the paired sequences I and II; III and IV; or V and VI run contiguously with each other without intervening base pairs between the two, in each case. These contiguous sequences are designated sequence VII, VIII and IX respectively.

For the purpose of expressing SEFA it will be realised by the skilled man that all the sequences above may comprise degenerate codons instead of those listed above. It is not envisaged that such use will necessarily provide any advantage as preparation would be probably be more lengthy, but some transformed microorganisms may express SEFA more readily with certain codons in degenerate form suited to them.

The present invention provides novel recombinant plasmids, comprising the recombinant DNA comprising either paired sequences selected from I and II, III and IV, or V and VI or the contiguous sequences VII, VIII and IX, the degenerative or allelic equivalents of any of these; said plasmids being capable of expressing polypeptides characteristic of SEFA when used to transform suitable microorganisms.

These recombinant plasmids may then be used to transform a host, such as E coli or yeast, whereby use of cloning and selection methods provides clones which contain the particular sequence or suitably flanked antigen encoding portion having expression enabling sequences with it. Convenient tools for the selection of these clones are the aforementioned sequences themselves as modified in known ways to provide probes, ie. by radiolabelling. Such probe sequences are readily provided by use of the polymerase chain reaction on native SEFA sequence template or by DNA synthesizer techniques;
radiolabelling being achieved using standard techiques to tag on ³²P.

Preferred microorganisms for transformation are E. coli and yeasts; a particularly preferred microorganism being E. coli DH5alpha. Thus preferred plasmids will be those known to the man skilled in the art as suitable for transforming such organisms. Particularly preferred plasmids are accordingly pBR322, pACYC184 and, most preferred, pUC18.

The polynucleotides sequences above may be combined with any of these known plasmids for the purposes of providing the novel plasmids of the invention. Particularly preferred will be plasmids into which polynucleotides consisting of the contiguous sequences VIII or IX have been inserted as these will be readily provided from cultured S. enteritidis or S. dublin by use of restriction endonucleases and encode for the entire SEFA amino acid sequence.

In this respect use of antibodies targeted for SEFA allows facile recognition of transformed organisms which is particularly useful for selecting expressing organisms from a background population. Such antibodies are the subject of copending MAFF patent application (PCT GB 91/01690 of inventor C J Thorns). That application discusses the use of two different monoclonal antibodies targeted at SEFA, designated MAB69/25 and 71/3, these antibodies are excreted by the hybrodoma cell lines deposited at the ECACC under the accession numbers 90101101 and 90121902 respectively. MAB69/25 is employed in Tables I and II.

For example, the contiguous sequence IX may be blunt-ended using Klenow polymerase infilling and then ligated into a plasmid such as pUC18. Alternatively total genomic DNA is extracted from S. enteritidis or a strain of S. dublin possessing said fimbrial antigen, as determined using the monoclonal antibodies and techniques disclosed in the applicants copending application referred to above, and then partially digested with SauIIIA restriction endonuclease to leave large fragments, some of which contain the sequences referred to above, which are then ligated into the plasmid vectors above.

The vectors of the present invention have further utility in so far as the contiguous sequences VII, VIII and IX all comprise a single BamH1 restriction endonuclease recognition site into which foreign peptide encoding DNA may be ligated by which it is sited within the reading frame of the transformant transcription system. This site is at the junction between the two sequences that make up the contiguous sequence; that occuring between base pair 255 and 256 in the numbering system applied at the bottom of each 10 base pairs above. Thus the present invention provides plasmids and transformants comprising the sequences I and II, or III and IV, or V and VI, or their degenerative or allelic equivalents, which have been augmented with further sequences. The invention provides a method for preparing these plasmid and transformants which inserts the further sequences into plasmids comprising the contiguous sequences VII, VIII or IX the at that BamH1 site.

Such augmented transformants are potentially capable of expression of mixed epitopic polypeptides comprising epitopes of SEFA together with further 'foreign' peptides. This opens the way to recombinantly produced peptides that are not easily expressed by other means. The fact that SEFA is a polypeptide that is passed to the exterior of the Salmonella cell of advantage in the recovery of such expressed polypeptides. The 'foreign' peptides may be further SEFA epitopes such as are bound by the antibodies above.

Thus the invention also provides micro-organisms, eg E.coli or yeasts, which have been transformed by insertion of one or more of the aforementioned sequences eg, by use of said plasmids.

Use of the micro-organisms provided by the invention gives a method of expression of the antigenic amino acid sequence SEFA referred to above and epitopic parts thereof which might be used as antigenic activity, that is having the ability to evoke production of antibodies in animal bodies.

In addition to use of the transformant expressed SEFA or epitopic parts thereof for immunological test purposes and kits for such, the recognition of the significance of the DNA sequences defined above provides methods of determination of DNA or RNA as being derived from the S. enteritidis or S. dublin serotypes in other, DNA/RNA based, tests.

**TABLE I**

| 264 Salmonella strains examined with monoclonal antibody MAB69/25 | | | |
|---|---|---|---|
| Serogroup Serotype (No. strains tested) | | Serogroup Serotype (No. strains tested) | |
| B | S. agama (1) | D1 | S. gallinarium (44) |
| | S. agona (1) | | S. moscow (1) |
| | S. bredeney (1) | | S. ouakam (1) |
| | S. derby (1) | | S. panama (1) |
| | S. heidelberg (1) | | S. pullorum (3) |
| | S. indiana (1) | | S. wangata (1) |
| | S. reading (1) | E1 | S. anatum (1) |
| | S. schwarzengrund (1) | | S. give (1) |
| | S. stanley (1) | | S. lexington (1) |
| | S. typhimurium (64) | | S. london (1) |
| C1 | S. bareilly (1) | | S. meleagridis (1) |
| | S. infantis (1) | | S. nchanga (1) |
| | S. lille (1) | | S. orion (1) |
| | S. livingstone (1) | E2 | S. binza (1) |
| | S. mbandaka (1) | | S. drypool (1) |
| | S. montevideo (1) | | S. manila (1) |
| | S. ohio (1) | | S. newington (1) |
| | S. oranienburg (1) | E4 | S. taksony (1) |
| | S. oslo (1) | | S. senftenberg (1) |
| | S. thompson (1) | F | S. aberdeen (1) |
| | S. virchow (1) | G1 | S. havana (1) |
| C2 | S. goldcoast (1) | | S. worthington (1) |
| | S. hadar (1) | G2 | S. ajiobo (1) |
| | S. newport (1) | | S. kedougou (1) |
| C3 | S. albany (1) | K | S. cerro (1) |
| | S. kentucky (2) | N | S. urbana (1) |
| | S. tado (1) | O | S. adelaide (1) |
| D1 | S. berta (1) | | S. ealing (1) |
| | S. canastel (1) | R | S. johannesburg (1) |
| | S. dublin (36) | S | S. offa (1) |
| | S. durban (1) | T | S. gera (1) |
| | S. enteritidis (58) | | |

**TABLE II**

| Direct binding of MAB 69/25 to Salmonella strains | | | |
|---|---|---|---|
| Serotype | | Number Examined | Monoclonal antibody MAB 69/25 %bound |
| S. enteritidis | PT 1 | 2 | 56^{a}(48-64)^{b} |
| S. enteritidis | PT 4 | 22 | 57 (14-100) |
| S. enteritidis | PT 4 plasmid minus | 6 | 57 (49-65) |
| S. enteritidis | PT 5 | 1 | 83 |
| S. enteritidis | PT 6 | 1 | 57 |
| S. enteritidis | PT 7 | 1 | 89 (85-93) |
| S. enteritidis | PT 8 | 12 | 53 (15-90) |
| S. enteritidis | PT 9 | 4 | 20 (17-23) |
| S. enteritidis | PT 11 | 7 | 50 (23-77) |
| S. enteritidis | PT 30 | 1 | 15 |
| S. enteritidis | untypable | 1 | 41 |
| S. dublin | | 12 | 25 (9-40) |
| S. dublin | | 24 | 0 |
| S. moscow | | 1 | 9 |
| Other Salmonella strains^{c} | | 169 | 0 |

| | | | |
|---|---|---|---|
| ^{a} Mean percentage of antibody binding relative to binding to high control (see text) | | | |
| ^{b} Range of binding | | | |
| ^{c} Serotypes listed in Table I PT = Phage type | | | |

The present invention further provides methods for determining the presence of microorganisms having DNA or RNA polynucleotide sequence encoding for SEFA or an epitopic part thereof, or such DNA or RNA itself, comprising:
(a) providing a sample suspected of containing said encoding polynucleotide sequence;
(b) determining the presence of said sequence by monitoring hybridization of SEFA targeted polynucleotide probes to it.

Such hybridization technique is carried out by methods that are now conventional in the art, using probes which are comprised of sequences complementary to a significant part of the target sequence and using temperature conditions suitable to achieved a desired stringency dependent on the degree of match of the probe to the target.

In a preferred form of this method the invention further provides methods for determining the presence of microorganisms having DNA or RNA polynucleotide sequence encoding for SEFA or an epitopic part thereof, or such DNA or RNA itself, comprising:
(a) providing a sample suspected of containing said encoding polynucleotide sequence;
(b) subjecting said sample to conditions under which polynucleotide sequences comprising sequences (I) and (II) are replicated by use of the polymerase chain reaction;
(c) determining the presence of any sequence produced.

Conveniently the sequence produced is detected, in both cases, by use of a hybridization probe suitably specific thereto which comprises any of the aforementioned sequences, more specifically being one of the sequences in a suitably labelled form eg. being labelled in some way as will be known to a man skilled in the art. Most conveniently the label will incorporate radioactive phophorous (³²P). A preferred such method comprises a PCR step (b) which employs primer pairs comprising one primer selected from groups (A) and the other from group(B):

Group A:

A1: 5' -GTGCGAATGCTAATAGTTGA- 3'

A2: 5' -TGCGTAAATCAGCATCTGCA- 3'

A3: 5' -TCTGCAGTAGCAGTTCTTGC- 3'

A4: 5' -GCTCAGAATACAACATCAGCCAA- 3'

Group B:

B1: 5' -AAAACAGGCTGTCCTTGTCCA- 3'

B2: 5' -TTAGCGTTTCTTGAGAGCTGG- 3'

B3: 5' -TTTTGATACTGCTGAACGTAG- 3'

The primers are numbered A1 to A4 and B1 to B3 for the purposes of identification later in this specification.

Any of the possible pairs selected in this way will identify the characteristic sequences VI, VII or IX sufficiently specifically enough for serotype determination purposes, ie: for determination of a Salmonella as being a SEFA encoding serotype and thus of one of the serotypes listed above.

As will be understood by a man skilled in the art, sequences which will specifically hybridize with sequence (VII) will include sequence (VII) itself, those having 75% or more, preferably 90% or more conformity to that sequence, and sequences comprising either strand of the two complementary sequences of any of these. Thus the step (c) of the method of this aspect of the invention may be carried out using a variety of hybridization probes that combine sufficiently specifically with the characteristic 'target' sequence comprising sequence (VII). For most purposes the primer sequences selected from those of groups (A) and (B) will be sufficiently specific to give reliable determination of the characteristic sequence, especially if a different 'primer' sequence is used for the probe of step (c) than those used for step (b).

The step (b) is carried out using the enzyme Taq polymerase as is now conventional in the art. The necessary conditions are those as described in EP-A-0201184 or EP-A-0200362 (both Cetus Corpn.) In such reaction, the appropriate primers derived from the sequences act as initiators for synthesis of large quantities of DNA identical to, or substantially identical to the initial double stranded DNA sequence. In this way substantially larger quantities of the DNA sequence may be made from the small quantities which may be available by isolation from the S. enteritidis or S. dublin thus increasing the amount of sequence available to be detected. The mere opresence of increased amount of DNA may be used in this case to signify presence of target sequence.

The genetically transformed organisms of the invention and their use to produce SEFA and SEFA containing sequences of the invention will now be described by way of example only, the examples including use of the detection methods of the invention for confirming presence of transformants:

### Example A. Preparation and cloning of S. enteritidis fimbrial antigen genes.

Step A1. Total genomic DNA was extracted from S. enteritidis using the method described in J B Goldberg & D E Ohman, (1984) J Bact 158 1115-1121.
Step A2. The DNA from step A1 was partially digested with SauIIIA restriction endonuclease to yield fragments with an size range between 5 and 10 kb. 2ug of genomic DNA in a Tris-HCl based buffer of pH 7.4 were mixed with 0.25 units of SauIIIA and incubated at 37°C.
Step A3. Cloning vector pUC18 was digested to completion with BamH1, giving compatible cohesive ends with SauIIIA, and was dephosphorylated with calf intestinal phosphatase.
Step A4. S. enteritidis DNA was ligated with vector pUC18 using T4 DNA ligase supplied by Bethesda Research Laboratories Life Technologies Inc. (Cat. No. 5224SB/SC ). The supplier's instructions for use in ligation were followed.
Step A5. The recombinant plasmid from step A4 was used to transform commercially available E.coli DH5alpha supplied by Bethesda Labs (see above) as Library Efficiency (RTM) DH5alpha Competant Cells (Cat. No. 8263SA) using the supplier's instructions to produce a genomic library.
Step A6. Transformants were transferred to the surface of HYBOND-C filters by replica plating for Western Blotting. Standard Western Blotting procedures using the S. enteritidis fimbrial antigen specific monoclonal antibody MAB 69/25, derived by standard techniques from hybridoma cells deposited under Accession No.90101101 on 11 October 1990 at the European Collection of Animal Cell Cultures, PHLS Centre for Applied Microbiology & Research, Porton Down, Salisbury, Wiltshire SP4 OJG, United Kingdom, as described and claimed in copending application No (PCT GB91 ;our ref P0958WOD), were done to identify transformant colonies expressing SEFA and thus containing the aforementioned sequences (VI), (VII) and (IX).
Step A7. The recombinant plasmids from fimbrial antigen positive transformants were extracted and used in confirmatory tests to prove the insert encoded said fimbrial antigen.

At the end of stage A7 it is possible to probe the DNA of said transformants to show the presence of the sequences and then to analyse said sequence by known sequencing methods.

### EXAMPLE B: Presentation of epitopes within the SEFA antigen by insertion of foreign DNA, in frame, into the SEFA encoding sequence.

As stated above, the present invention further provides the prospect of exploitation of the polynucleotide sequences of the present invention having with sequences encoding for desired foreign protein or peptide products to produce transformants having ability to secrete the desired product.

SB10 epitope of Mycobacterium bovis secreted antigen, MPB70 (Radford et al. (1990), J. Gen. Micro, 136: 265-272) consists of the amino acid sequence as encoded for below:

Q   D   P   V          encoded amino acid

5'- CAG GAC CCG GTC -3'    coding/master strand
3'- GTC CTG GGC CAG -5'    complimentary strand

Synthetic oligonucleotides encompassing this sequence and providing BamH1 cohesive ends were made using an ABI PCR MATE EP model 391 DNA synthesizer following the manufacturer's methods. The oligonucleotides were as follows:

SB10.1 5'- GAT CAG GAC CCG GTC GCT -3'
SB10.2 3'- TC CTG GGC CAG CGA CTA G -5'

The two oligonucleotides, SB10.1 and SB10.2 were allowed to anneal to form a double stranded (duplex) molecule by heating to 95°C and then cooling to room temperature over a two hour period. Annealing was assessed by comparing rate of migration of the duplex molecule compared with the rate of migration of the two single oligonucleotides when run through 4% agarose in TBE buffer. A marginal retardation in migration rate was observed and suggested near 100% annealing.

A lambda EMBL library was prepared from S.enteritidis strain 1246 providing a 9 to 23 kilobase library which was probed with the SEFA sequence IX (consisting of sequences V and VI run contiguously). Hybridizing fragments were subcloned into pUC18 and a suitable vector comprising the SEFA antigen gene flanked by adjacent contiguous chromosomal DNA was selected on its ability to transform E.coliDH5 alpha to a SEFA expressing form: all general methods as conventional to the art (see eg. Maniatis).

The pUC18 vector so obtained was digested with BamH1 and agarose gel electrophoresis demonstrated that the DNA was cut once at the unique BamH1 site within the SEFA gene. Cut vector and duplex oligonucleotide (SB10.1 plus SB10.2) were mixed together (1:10 ratio) and ligated using T4 ligase (Life Technologies) using the manufacturers methods. The saturating amounts of duplex oligonucleotide increased rate of insertion and the lack of terminal phosphate groups on the duplex prevented multiple insertion. The ligated construct was designed to be as follows:

Q D Q D P V A D P amino acid

5'- CAG GAT CAG GAC CCG GTC GCT GAT CCT -3' coding/master strand
3'- GTC CTA GTC CTG GGC CAG CGA CTA GGA -5' complimentary strand

The ligated construct lacks the GGATCC BamH1 recognition sequence.

Thus prior to transforming the construct into E.coli DH5 alpha, the ligated DNA was cut with BamH1 to linearise any of the vector which lacked insert. The ligated DNA was then used to transform E.coli using standard procedures.

Recombinants were picked directly into a Polymerase Chain Reaction mixture in which the primers were designed to flank the insertion site to yield a product of 219 base pairs without insert or 237 base pairs with insert. PCR products were sized by gel lectrophoresis and those shown to be 237 base pairs were tested by digestion with BamH1 to ensure loss of the site.

A sample (8ul) was taken from the aqueous phase of the PCR reaction mixture and made 20ul by addition of HPLC grade water, X10 reaction buffer and 5U BamH1. The PCR product was digested for 3 hours at 37°C. Control experiments using the 219 base pair product were performed to demonstrate digestion. The entire reaction mixtures were loaded onto agarose gels and the DNA products resolved; those PCR products shown to be 237 base pairs did not cut with BamH1 giving evidence for insertion of the oligonucleotide duplex.

To confirm the presence of the insert and determine its orientation, PCR experiments were set up in which the primers were SB10.2 and a series of primers from primer group A above (see page 18) toward the proximal (5') end of the SEFA antigen gene. Of twelve recombinants tested, five gave the desired sized product and were, therefore, shown to have the insert in the correct orientation.

To confirm that the insert was encoding the SB10 epitope and was 'in frame' with the SEFA antigen sequence, double stranded DNA sequencing using standard protocols was done on the five positive clones identified above. The primers used were:

5'- TCTGCAGTAGCAGTTCTTGC -3' for the coding strand and

5'- AAAACAGGCTGTCCTTGTCCA -3' for the complimentary strand.

The DNA sequence of both strands across the insert site was established and was as predicted above.

E. coli recombinants harbouring the constructs, designated SEFA::SB10. 1 to 5 were tested immunologically for the production of SEFA. Western blots of whole E. coli cells harbouring each of the SEFA::SB10 constructs demonstrated the presence of a protein of about 15kDal (and a less intense protein band of about 18.5 kDal) when using anti-SEFA polyclonal and anti-SEFA monoclonal antibody 69/25. In control experiments, E. coli recombinants harbouring the vector gave protein bands of 14.5kDal and 18kDal in Western blot experiments using the same antibodies.

This data clearly demonstrates that the SEFA polynucleotide sequence may be modified to express additional amino acids within its primary structure without the loss of reactivity to one SEFA epitope specific antibody.

The complete sequence of the largest of the sequences of the invention, sequence IX, is given below with the sequences I, II, III, IV, V, VI, VII and VIII being indicated together with the probe sequences from probe groups A and B. These sequences are marked by reference to their 5' and 3' ends: eg. I-5', I-3' etc. The numbering given below each 10 base pairs of the sequences to VI above being related to their positions in this sequence IX.

Sequence IX

V-5'
5'- GATCCTTGTT TTTTTTCTTA AATTTTTAAA ATGGCGTGAG TATATTAGCA TCCGCACAGA
3'- CTAGGAACAA AAAAAAGAAT TTAAAAATTT TACCGCACTC ATATAATCGT AGGCGTGTCT
10 20 30 40 50 60

A1-5' III-5' A1-3' A2-5'
TAAATTGTGC GAATGCTAAT AGTTGATTTT TGGAGATTTT GTAATATGCG TAAATCAGCA
ATTTAACACG CTTACGATTA TCAACTAAAA ACCTCTAAAA CATTATACGC ATTTAGTCGT
70 80 90 100 110 120
A3-5'
A2-3' A3-3'
TCTGCAGTAG CAGTTCTTGC TTTAATTGCA TGTGGCAGTG CCCACGCAGC TGGCTTTGTT
AGACGTCATC GTCAAGAACG AAATTAACGT ACACCGTCAC GGGTGCGTCG ACCGAAACAA
130 140 150 160 170 180

A4-5'
GGTAACAAAG CAGAGGTTCA GGCAGCGGTT ACTATTGCAG CTCAGAATAC AACATCAGCC
CCATTGTTTC GTCTCCAAGT CCGTCGCCAA TGATAACGTC GAGTCTTATG TTGTAGTCGG
190 200 210 220 230 240
A4-3'
I,III and V -3':II,IV and VI-5'
AACTGGAGTC AGGATCCTGG CTTTACAGGG CCTGCTGTTG CTGCTGGTCA GAAAGTTGGT
TTGACCTCAG TCCTAGGACC GAAATGTCCC GGACGACAAC GACGACCAGT CTTTCAACCA
250 260 270 280 290 300
BamH1 site.

ACTCTCAGCA TTACTGCTAC TGGTCCACAT AACTCAGTAT CTATTGCAGG TAAAGGGGCT
TGAGAGTCGT AATGACGATG ACCAGGTGTA TTGAGTCATA GATAACGTCC ATTTCCCCGA
310 320 330 340 350 360

II-3'
TCGGTATCTG GTGGTGTAGC CACTGTCCCG TTCGTTGATG GACAAGGACA GCCTGTTTTC
AGCCATAGAC CACCACATCG GTGACAGGGC AAGCAACTAC CTGTTCCTGT CGGACAAAAG
370 380 390 400 410 420
B1-3' B1-5'

CGTGGGCGTA TTCAGGGAGC CAATATTAAT GACCAAGCAA ATACTGGAAT TGACGGGCTT
GCACCCGCAT AAGTCCCTCG GTTATAATTA CTGGTTCGTT TATGACCTTA ACTGCCCGAA
430 440 450 460 470 480

GCAGGTTGGC GAGTTGCCAG CTCTCAAGAA ACGCTAAATG TCCCTGTCAC AACCTTTGGT
CGTCCAACCG CTCAACGGTC GAGAGTTCTT TGCGATTTAC AGGGACAGTG TTGGAAACCA
490 500 510 520 530 540
B2-3' B2-5'

IV-3'
AAATCGACCC TGCCAGCAGG TACTTTCACT GCGACCTTCT ACGTTCAGCA GTATCAAAAC
TTTAGCTGGG ACGGTCGTCC ATGAAAGTGA CGCTGGAAGA TGCAAGTCGT CATAGTTTTG
550 560 570 580 590 600
B3-3' B3-5'

TAATTTAATT TAAACTTTAT AAATGCCCTC AATATGAGCG AGTTTGGATA ATTTTATTAT
ATTAAATTAA ATTTGAAATA TTTACGGGAG TTATACTCGC TCAAACCTAT TAAAATAATA
610 620 630 640 650 660

TTTAAAAATA TCTATTTTGA ATAGATAGGT TTTATGCTTC CATGCAAAAA CTTAAAGAGG
AAATTTTTAT AGATAAAACT TATCTATCCA AAATACGAAG GTACGTTTTT GAATTTCTCC
670 680 690 700 710 720

GATTATGTAT ATTTTGAATA AATTTATACG TAGAACTGTT ATCTTTTTCC TTTTTTTTGC
CTAATACATA TAAAACTTAT TTAAATATGC ATCTTGACAA TAGAAAAAGG AAAAAAAACG
730 740 750 760 770 780

TACCTTCCAA TTGCTTCTTC GGAAAGTAAA AAAATTGAGC AACCATTATT AACACAAAAA
ATGGAAGGTT AACGAAGAAG CCTTTCATTT TTTTAACTCG TTGGTAATAA TTGTGTTTTT
790 800 810 820 830 840

TATTATGGCC TAAGATTGGG CACTACACGT GTTATTTATA AAGAAGATGC TCCATCAACA
ATAATACCGG ATTCTAACCC GTGATGTGCA CAATAAATAT TTCTTCTACG AGGTAGTTGT
850 860 870 880 890 900

AGTTTTTGGA TTATGAATGA AAAAGAATAT CCAATCCTTG TTCAAACTCA AGTATATAAT
TCAAAAACCT AATACTTACT TTTTCTTATA GGTTAGGAAC AAGTTTGAGT TCATATATTA
910 920 930 940 950 960

GATGATAAAT CATCAAAAGC TCCATTTATT GTAACACCAC CTATTTTGAA AGTTGAAAGT
CTACTATTTA GTAGTTTTCG AGGTAAATAA CATTGTGGTG GATAAAACTT TCAACTTTCA
970 980 990 1000 1010 1020

AATGCGCGAA CAAGATTGAA GGTAATACCA ACAAGTAATC TATTCAATAA AAATGAGGAG
TTACGCGCTT GTTCTAACTT CCATTATGGT TGTTCATTAG ATAAGTTATT TTTACTCCTC
1030 1040 1050 1060 1070 1080

TCTTTGTATT GGTTGTGTGT AAAAGGAGTC CCACCACTAA ATGATAATGA AAGCAATAAT
AGAAACATAA CCAACACACA TTTTCCTCAG GGTGGTGATT TACTATTACT TTCGTTATTA
1090 1100 1110 1120 1130 1140

AAAAACAACA TAACTACGAA TCTTAATGTG AATGTGGTTA CGAATAGTTG TATTAAATTA
TTTTTGTTGT ATTGATGCTT AGAATTACAC TTACACCAAT GCTTATCAAC ATAATTTAAT
1150 1160 1170 1180 1190 1200

ATTTATAGGC CTAAAACTAT AGACTTAACG ACAATGGAGA TTGCAGATAA ATTAAAGTTA
TAAATATCCG GATTTTGATA TCTGAATTGC TGTTACCTCT AACGTCTATT TAATTTCAAT
1210 1220 1220 1240 1250 1260

GAGAGAAAAG GAAATAGTAT AGTTATAAAG AATCCAACAT CATCATATGT GAATATTGCA
CTCTCTTTTC CTTTATCATA TCAATATTTC TTAGGTTGTA GTAGTATACA CTTATAACGT
1270 1280 1290 1300 1310 1320

AATATTAAAT CTGGTAATTT AAGTTTTAAT ATTCCAAATG GATATATTGA GCCATTTGGA
TTATAATTTA GACCATTAAA TTCAAAATTA TAAGGTTTAC CTATATAACT CGGTAAACCT
1330 1340 1350 1360 1370 1380

TATGCTCAAT TACCTGGTGG AGTACATAGT AAAATAACTT TGACTATTTT GGATGATAAC
ATACGAGTTA ATGGACCACC TCATGTATCA TTTTATTGAA ACTGATAAAA CCTACTATTG
1390 1400 1410 1420 1430 1440

GGCGCTGAAA TTATAAGAGA ATTATTAGTT TAAGGTGTAA AACAAATGAA GAAAACCACA
CCGCGACTTT AATATTCTCT TAATAATCAA ATTCCACATT TTGTTTACTT CTTTTGGTGT
1450 1460 1470 1480 1490 1500

ATTACTCTAT TTGTTTTAAC CAGTGTATTT CACTCTGGAA ATGTTTTCTC CAGACAATAT
TAATGAGATA AACAAAATTG GTCACATAAA GTGAGACCTT TACAAAAGAG GTCTGTTATA
1510 1520 1530 1540 1550 1560

AATTTCGACT ATGGAAGTTT GAGTCTTCTC CCGGTGAGAA TGCATCTTTT CTAAGTGTTG
TTAAAGCTGA TACCTTCAAA CTCAGAAGAG GGCCACTCTT ACGTAGAAAA GATTCACAAC
1570 1580 1590 1600 1610 1620

AAACGCTTCC CTGGTAATTA TGTTGTTGAT GTATATTTGA ATAATCAGTT AAAAGAAACT
TTTGCGAAGG GACCATTAAT ACAACAACTA CATATAAACT TATTAGTCAA TTTTCTTTGA
1630 1640 1650 1660 1670 1680

ACTGAGTTGT ATTTCAAATC AATGACTCAG ACTCTAGAAC CATGCTTAAC AAAAGAAAAA
TGACTCAACA TAAAGTTTAG TTACTGAGTC TGAGATCTTG GTACGAATTG TTTTCTTTTT
1690 1700 1710 1720 1730 1740

CTTATAAAGT ATGGGATCGC CATCCAGGAG CTTCATGGGT TGCAGTTTGA TAATGAACAA
GAATATTTCA TACCCTAGCG GTAGGTCCTC GAAGTACCCA ACGTCAAACT ATTACTTGTT
1750 1760 1770 1780 1790 1800

TGCGTTCTCT TAGAGCATTC TCCTCTTTAA ATATACTTAT AACGCGGCTA ACCAAAGTTT
ACGCAAGAGA ATCTCGTAAG AGGAGAAATT TATATGAATA TTGCGCCGAT TGGTTTCAAA
1810 1820 1830 1840 1850 1860

GCTTTTAAAT GCACCATCTA AAATTCTATC TCCAATAGAC AGTGAAATTG CTGATGAAAA
CGAAAATTTA CGTGGTAGAT TTTAAGATAG AGGTTATCTG TCACTTTAAC GACTACTTTT
1870 1880 1890 1900 1910 1920

TATCTGGGAT GATGGCATTA ACGCTTTTCT TTTAAATTAC AGAGCTTAAT TATTTGCATT
ATAGACCCTA CTACCGTAAT TGCGAAAAGA AAATTTAATG TCTCGAATTA ATAAACGTAA
1930 1940 1950 1960 1970 1980

CTAAGGTTGG AGGAGAGAGA TTCATACTTT GGTCAAATTC AACCTTGGTT TTAATTTTGG
GATTCCAACC TCCTCTCTCT AAGTATGAAA CCAGTTTAAG TTGGAACCAA AATTAAAACC
1990 2000 2010 2020 2030 2040

TCCCTGGCGG CTAAGGAATC TATCATCTTG GCAAAACTTG TCAAGCGAAA AAAAATTTGA
AGGGACCGCC GATTCCTTAG ATAGTAGAAC AGTTTTGAAC AGTTCGCTTT TTTTTAAACT
2050 2060 2070 2080 2090 2100

ATCAGCATAT ATTTATGCTG AGCGAGGTTT AAAAAAAATA AAGAGCAAAC TAACAGTTGG
TAGTCGTATA TAAATACGAC TCGCTCCAAA TTTTTTTTAT TTCTCGTTTG ATTGTCAACC
2110 2120 2130 2140 2150 2160

GGACAAATAT ACCAGTGCAG ATTTATTCGA TAGCGTACCA TTTAGAGGCT TTTCTTTAAA
CCTGTTTATA TGGTCACGTC TAAATAAGCT ATCGCATGGT AAATCTCCGA AAAGAAATTT
2170 2180 2190 2200 2210 2220

TAAAGATGAA AGTATGATAC CTTTCTCACA GAGAACATAT TATCCAACAA TACGTGGTAT
ATTTCTACTT TCATACTATG GAAAGAGTGT CTCTTGTATA ATAGGTTGTT ATGCACCATA
2230 2240 2250 2260 2270 2280

TGCGAAAACC AATGCGACTG TAGAAGTAAG ACAAAATGGA TACTTGATAT ATTCTACTTC
ACGCTTTTGG TTACGCTGAC ATCTTCATTC TGTTTTACCT ATGAACTATA TAAGATGAAG
2290 2300 2310 2320 2330 2340

VI-3'
AGTCCCCCCC GGGCAATTCG AGATAGGTAG AGAACAAATT GCTGATC -3'
TCAGGGGGGG CCCGTTAAGC TCTATCCATC TCTTGTTTAA CGACTAG -5'
2350 2360 2370 2380

## Claims

1. Recombinant DNA encoding
(a) the Salmonella enteritidis fimbrial antigen (SEFA) amino acid sequence: 
M L I V D F W R F C N M R K S A S A V A V L A L I A C G S A H A A G F
V G N K A E V Q A A V T I A A Q N T T S A N W S Q D P G F T G P A V A
A G Q K V G T L S I T A T G P H N S V S I A G K G A S V S G G V A T V
P F V D G Q G Q P V F R G R I Q G A N I N D Q A N T G I D G L A G W R
V A S S Q E T L N V P V T T F G K S T L P A G T F T A T F Y V Q Q Y Q
N
(b) an epitopic part thereof, or
(c) an allelic variant of either
the epitopic part and the allelic variant being characterised in that they are capable of specific binding with monoclonal antibody secreted by at least one of the hybridoma cell lines deposited at the ECACC under the accession numbers 90101101 and 90121902.

2. Recombinant DNA as claimed claim 1 wherein suitable flanking sequences for control of amino acid sequence expression are provided.

3. Recombinant DNA as claimed in claim 1 or claim 2 comprising the Sequences I and II: 
Sequence I

5'- G CTCAGAATAC AACATCAGCC AACTGGAGTC AGGAT -3'
3'- C GAGTCTTATG TTGTAGTCGG TTGACCTCAG TCCTA -5'
230 240 250
 
Sequence II

5'- CCTGG CTTTACAGGG CCTGCTGTTG CTGCTGGTCA GAAAGTTGGT
3'- GGACC GAAATGTCCC GGACGACAAC GACGACCAGT CTTTCAACCA
260 270 280 290 300

ACTCTCAGCA TTACTGCTAC TGGTCCACAT AACTCAGTAT CTATTGCAGG TAAAGGGGCT
TGAGAGTCGT AATGACGATG ACCAGGTGTA TTGAGTCATA GATAACGTCC ATTTCCCCGA
310 320 330 340 350 360

TCGGTATCTG GTGGTGTAGC CACTGTCCCG TTCGTTGATG GACAAGGACA GCCTGTTTT -3'
AGCCATAGAC CACCACATCG GTGACAGGGC AAGCAACTAC CTGTTCCTGT CGGACAAAA -5'
370 380 390 400 410
 or sequences degenerately equivalent thereto.

4. Recombinant DNA as claimed in any one of the preceding claims comprising Sequences III and IV: 
Sequence III

5'- ATGCTAAT AGTTGATTTT TGGAGATTTT GTAATATGCG TAAATCAGCA
3'- TACGATTA TCAACTAAAA ACCTCTAAAA CATTATACGC ATTTAGTCGT
80 90 100 110 120

TCTGCAGTAG CAGTTCTTGC TTTAATTGCA TGTGGCAGTG CCCACGCAGC TGGCTTTGTT
AGACGTCATC GTCAAGAACG AAATTAACGT ACACCGTCAC GGGTGCGTCG ACCGAAACAA
130 140 150 160 170 180

GGTAACAAAG CAGAGGTTCA GGCAGCGGTT ACTATTGCAG CTCAGAATAC AACATCAGCC
CCATTGTTTC GTCTCCAAGT CCGTCGCCAA TGATAACGTC GAGTCTTATG TTGTAGTCGG
190 200 210 220 230 240
 
AACTGGAGTC AGGAT -3'
TTGACCTCAG TCCTA -5'
250
 
Sequence IV

5'- CCTGG CTTTACAGGG CCTGCTGTTG CTGCTGGTCA GAAAGTTGGT
3'- GGACC GAAATGTCCC GGACGACAAC GACGACCAGT CTTTCAACCA
260 270 280 290 300

ACTCTCAGCA TTACTGCTAC TGGTCCACAT AACTCAGTAT CTATTGCAGG TAAAGGGGCT
TGAGAGTCGT AATGACGATG ACCAGGTGTA TTGAGTCATA GATAACGTCC ATTTCCCCGA
310 320 330 340 350 360

TCGGTATCTG GTGGTGTAGC CACTGTCCCG TTCGTTGATG GACAAGGACA GCCTGTTTTC
AGCCATAGAC CACCACATCG GTGACAGGGC AAGCAACTAC CTGTTCCTGT CGGACAAAAG
370 380 390 400 410 420

CGTGGGCGTA TTCAGGGAGC CAATATTAAT GACCAAGCAA ATACTGGAAT TGACGGGCTT
GCACCCGCAT AAGTCCCTCG GTTATAATTA CTGGTTCGTT TATGACCTTA ACTGCCCGAA
430 440 450 460 470 480

GCAGGTTGGC GAGTTGCCAG CTCTCAAGAA ACGCTAAATG TCCCTGTCAC AACCTTTGGT
CGTCCAACCG CTCAACGGTC GAGAGTTCTT TGCGATTTAC AGGGACAGTG TTGGAAACCA
490 500 510 520 530 540

AAATCGACCC TGCCAGCAGG TACTTTCACT GCGACCTTCT ACGTTCAGCA GTATCAAAAC -3'
TTTAGCTGGG ACGGTCGTCC ATGAAAGTGA CGCTGGAAGA TGCAAGTCGT CATAGTTTTG -5'
550 560 570 580 590 600
 or sequences degenerately equivalent thereto.

5. Recombinant DNA as claimed in any one of the preceding claims comprising Sequences V and VI 
Sequence V

5'- GATCCTTGTT TTTTTTCTTA AATTTTTAAA ATGGCGTGAG TATATTAGCA TCCGCACAGA
3'- CTAGGAACAA AAAAAAGAAT TTAAAAATTT TACCGCACTC ATATAATCGT AGGCGTGTCT
10 20 30 40 50 60

TAAATTGTGC GAATGCTAAT AGTTGATTTT TGGAGATTTT GTAATATGCG TAAATCAGCA
ATTTAACACG CTTACGATTA TCAACTAAAA ACCTCTAAAA CATTATACGC ATTTAGTCGT
70 80 90 100 110 120

TCTGCAGTAG CAGTTCTTGC TTTAATTGCA TGTGGCAGTG CCCACGCAGC TGGCTTTGTT
AGACGTCATC GTCAAGAACG AAATTAACGT ACACCGTCAC GGGTGCGTCG ACCGAAACAA
130 140 150 160 170 180

GGTAACAAAG CAGAGGTTCA GGCAGCGGTT ACTATTGCAG CTCAGAATAC AACATCAGCC
CCATTGTTTC GTCTCCAAGT CCGTCGCCAA TGATAACGTC GAGTCTTATG TTGTAGTCGG
190 200 210 220 230 240

AACTGGAGTC AGGAT -3'
TTGACCTCAG TCCTA -5'
250
 
Sequence VI

5'- CCTGG CTTTACAGGG CCTGCTGTTG CTGCTGGTCA GAAAGTTGGT
3'- GGACC GAAATGTCCC GGACGACAAC GACGACCAGT CTTTCAACCA
260 270 280 290 300
 
ACTCTCAGCA TTACTGCTAC TGGTCCACAT AACTCAGTAT CTATTGCAGG TAAAGGGGCT
TGAGAGTCGT AATGACGATG ACCAGGTGTA TTGAGTCATA GATAACGTCC ATTTCCCCGA
310 320 330 340 350 360

TCGGTATCTG GTGGTGTAGC CACTGTCCCG TTCGTTGATG GACAAGGACA GCCTGTTTTC
AGCCATAGAC CACCACATCG GTGACAGGGC AAGCAACTAC CTGTTCCTGT CGGACAAAAG
370 380 390 400 410 420

CGTGGGCGTA TTCAGGGAGC CAATATTAAT GACCAAGCAA ATACTGGAAT TGACGGGCTT
GCACCCGCAT AAGTCCCTCG GTTATAATTA CTGGTTCGTT TATGACCTTA ACTGCCCGAA
430 440 450 460 470 480

GCAGGTTGGC GAGTTGCCAG CTCTCAAGAA ACGCTAAATG TCCCTGTCAC AACCTTTGGT
CGTCCAACCG CTCAACGGTC GAGAGTTCTT TGCGATTTAC AGGGACAGTG TTGGAAACCA
490 500 510 520 530 540

AAATCGACCC TGCCAGCAGG TACTTTCACT GCGACCTTCT ACGTTCAGCA GTATCAAAAC
TTTAGCTGGG ACGGTCGTCC ATGAAAGTGA CGCTGGAAGA TGCAAGTCGT CATAGTTTTG
550 560 570 580 590 600

TAATTTAATT TAAACTTTAT AAATGCCCTC AATATGAGCG AGTTTGGATA ATTTTATTAT
ATTAAATTAA ATTTGAAATA TTTACGGGAG TTATACTCGC TCAAACCTAT TAAAATAATA
610 620 630 640 650 660

TTTAAAAATA TCTATTTTGA ATAGATAGGT TTTATGCTTC [?]ATGCAAAA CTTAAAGAGG
AAATTTTTAT AGATAAAACT TATCTATCCA AAATACGAAG GTACGTTTTT GAATTTCTCC
670 680 690 700 710 720
 
GATTATGTAT ATTTTGAATA AATTTATACG TAGAACTGTT ATCTTTTTCC TTTTTTTTGC
CTAATACATA TAAAACTTAT TTAAATATGC ATCTTGACAA TAGAAAAAGG AAAAAAAACG
730 740 750 760 770 780

TACCTTCCAA TTGCTTCTTC GGAAAGTAAA AAAATTGAGC AACCATTATT AACACAAAAA
ATGGAAGGTT AACGAAGAAG CCTTTCATTT TTTTAACTCG TTGGTAATAA TTGTGTTTTT
790 800 810 820 830 840

TATTATGGCC TAAGATTGGG CACTACACGT GTTATTTATA AAGAAGATGC TCCATCAACA
ATAATACCGG ATTCTAACCC GTGATGTGCA CAATAAATAT TTCTTCTACG AGGTAGTTGT
850 860 870 880 890 900

AGTTTTTGGA TTATGAATGA AAAAGAATAT CCAATCCTTG TTCAAACTCA AGTATATAAT
TCAAAAACCT AATACTTACT TTTTCTTATA GGTTAGGAAC AAGTTTGAGT TCATATATTA
910 920 930 940 950 960

GATGATAAAT CATCAAAAGC TCCATTTATT GTAACACCAC CTATTTTGAA AGTTGAAAGT
CTACTATTTA GTAGTTTTCG AGGTAAATAA CATTGTGGTG GATAAAACTT TCAACTTTCA
970 980 990 1000 1010 1020

AATGCGCGAA CAAGATTGAA GGTAATACCA ACAAGTAATC TATTCAATAA AAATGAGGAG
TTACGCGCTT GTTCTAACTT CCATTATGGT TGTTCATTAG ATAAGTTATT TTTACTCCTC
1030 1040 1050 1060 1070 1080

TCTTTGTATT GGTTGTGTGT AAAAGGAGTC CCACCACTAA ATGATAATGA AAGCAATAAT
AGAAACATAA CCAACACACA TTTTCCTCAG GGTGGTGATT TACTATTACT TTCGTTATTA
1090 1100 1110 1120 1130 1140
 
AAAAACAACA TAACTACGAA TCTTAATGTG AATGTGGTTA CGAATAGTTG TATTAAATTA
TTTTTGTTGT ATTGATGCTT AGAATTACAC TTACACCAAT GCTTATCAAC ATAATTTAAT
1150 1160 1170 1180 1190 1200

ATTTATAGGC CTAAAACTAT AGACTTAACG ACAATGGAGA TTGCAGATAA ATTAAAGTTA
TAAATATCCG GATTTTGATA TCTGAATTGC TGTTACCTCT AACGTCTATT TAATTTCAAT
1210 1220 1220 1240 1250 1260

GAGAGAAAAG GAAATAGTAT AGTTATAAAG AATCCAACAT CATCATATGT GAATATTGCA
CTCTCTTTTC CTTTATCATA TCAATATTTC TTAGGTTGTA GTAGTATACA CTTATAACGT
1270 1280 1290 1300 1310 1320

AATATTAAAT CTGGTAATTT AAGTTTTAAT ATTCCAAATG GATATATTGA GCCATTTGGA
TTATAATTTA GACCATTAAA TTCAAAATTA TAAGGTTTAC CTATATAACT CGGTAAACCT
1330 1340 1350 1360 1370 1380

TATGCTCAAT TACCTGGTGG AGTACATAGT AAAATAACTT TGACTATTTT GGATGATAAC
ATACGAGTTA ATGGACCACC TCATGTATCA TTTTATTGAA ACTGATAAAA CCTACTATTG
1390 1400 1410 1020 1430 1440

GGCGCTGAAA TTATAAGAGA ATTATTAGTT TAAGGTGTAA AACAAATGAA GAAAACCACA
CCGCGACTTT AATATTCTCT TAATAATCAA ATTCCACATT TTGTTTACTT CTTTTGGTGT
1450 1460 1470 1480 1490 1500

ATTACTCTAT TTGTTTTAAC CAGTGTATTT CACTCTGGAA ATGTTTTCTC CAGACAATAT
TAATGAGATA AACAAAATTG GTCACATAAA GTGAGACCTT TACAAAAGAG GTCTGTTATA
1510 1520 1530 1540 1550 1560
 
AATTTCGACT ATGGAAGTTT GAGTCTTCTC CCGGTGAGAA TGCATCTTTT CTAAGTGTTG
TTAAAGCTGA TACCTTCAAA CTCAGAAGAG GGCCACTCTT ACGTAGAAAA GATTCACAAC
1570 1580 1590 1600 1610 1620

AAACGCTTCC CTGGTAATTA TGTTGTTGAT GTATATTTGA ATAATCAGTT AAAAGAAACT
TTTGCGAAGG GACCATTAAT ACAACAACTA CATATAAACT TATTAGTCAA TTTTCTTTGA
1630 1640 1650 1660 1670 1680

ACTGAGTTGT ATTTCAAATC AATGACTCAG ACTCTAGAAC CATGCTTAAC AAAAGAAAAA
TGACTCAACA TAAAGTTTAG TTACTGAGTC TGAGATCTTG GTACGAATTG TTTTCTTTTT
1690 1700 1710 1720 1730 1740

CTTATAAAGT ATGGGATCGC CATCCAGGAG CTTCATGGGT TGCAGTTTGA TAATGAACAA
GAATATTTCA TACCCTAGCG GTAGGTCCTC GAAGTACCCA ACGTCAAACT ATTACTTGTT
1750 1760 1770 1780 1790 1800

TGCGTTCTCT TAGAGCATTC TCCTCTTTAA ATATACTTAT AACGCGGCTA ACCAAAGTTT
ACGCAAGAGA ATCTCGTAAG AGGAGAAATT TATATGAATA TTGCGCCGAT TGGTTTCAAA
1810 1820 1830 1840 1850 1860

GCTTTTAAAT GCACCATCTA AAATTCTATC TCCAATAGAC AGTGAAATTG CTGATGAAAA
CGAAAATTTA CGTGGTAGAT TTTAAGATAG AGGTTATCTG TCACTTTAAC GACTACTTTT
1870 1880 1890 1900 1910 1920

TATCTGGGAT GATGGCATTA ACGCTTTTCT TTTAAATTAC AGAGCTTAAT TATTTGCATT
ATAGACCCTA CTACCGTAAT TGCGAAAAGA AAATTTAATG TCTCGAATTA ATAAACGTAA
1930 1940 1950 1960 1970 1980
 
CTAAGGTTGG AGGAGAGAGA TTCATACTTT GGTCAAATTC AACCTTGGTT TTAATTTTGG
GATTCCAACC TCCTCTCTCT AAGTATGAAA CCAGTTTAAG TTGGAACCAA AATTAAAACC
1990 2000 2010 2020 2030 2040

TCCCTGGCGG CTAAGGAATC TATCATCTTG GCAAAACTTG TCAAGCGAAA AAAAATTTGA
AGGGACCGCC GATTCCTTAG ATAGTAGAAC AGTTTTGAAC AGTTCGCTTT TTTTTAAACT
2050 2060 2070 2080 2090 2100

ATCAGCATAT ATTTATGCTG AGCGAGGTTT AAAAAAAATA AAGAGCAAAC TAACAGTTGG
TAGTCGTATA TAAATACGAC TCGCTCCAAA TTTTTTTTAT TTCTCGTTTG ATTGTCAACC
2110 2120 2130 2140 2150 2160

GGACAAATAT ACCAGTGCAG ATTTATTCGA TAGCGTACCA TTTAGAGGCT TTTCTTTAAA
CCTGTTTATA TGGTCACGTC TAAATAAGCT ATCGCATGGT AAATCTCCGA AAAGAAATTT
2170 2180 2190 2200 2210 2220

TAAAGATGAA AGTATGATAC CTTTCTCACA GAGAACATAT TATCCAACAA TACGTGGTAT
ATTTCTACTT TCATACTATG GAAAGAGTGT CTCTTGTATA ATAGGTTGTT ATGCACCATA
2230 2240 2250 2260 2270 2280

TGCGAAAACC AATGCGACTG TAGAAGTAAG ACAAAATGGA TACTTGATAT ATTCTACTTC
ACGCTTTTGG TTACGCTGAC ATCTTCATTC TGTTTTACCT ATGAACTATA TAAGATGAAG
2290 2300 2310 2320 2330 2340

AGTCCCCCCC GGGCAATTCG AGATAGGTAG AGAACAAATT GCTGATC -3'
TCAGGGGGGG CCCGTTAAGC TCTATCCATC TCTTGTTTAA CGACTAG -5'
2350 2360 2370 2380
 or sequences degenerately equivalent thereto.

6. Recombinant DNA as claimed in Claim 3 wherein the Sequences I and II are comprised within a contiguous sequence.

7. Recombinant DNA as claimed in Claim 4 wherein the Sequences III and IV are comprised within a contiguous sequence.

8. Recombinant DNA as claimed in Claim 5 wherein the Sequences V and VI are comprised within a contiguous sequence.

9. Recombinant DNA as claimed in any one of claims 1 to 5 further comprising a sequence encoding for a further amino acid sequence.

10. Recombinant DNA as claimed in claim 9 wherein the further amino acid sequence comprises additional epitopic parts of SEFA, said epitopic parts being characterised in that they are capable of specific binding with monoclonal antibody secreted by at least one of the hybridoma cell lines deposited at the ECACC under the accession numbers 90101101 and 90121902.

11. Recombinant DNA as claimed in claim 9 wherein the further amino acid sequence comprises a non-SEFA epitopic sequence.

12. Recombinant DNA as claimed in claim 11 wherein the non-SEFA epitopic sequence comprises SB10 epitope of Mycobacterium bovis.

13. A novel plasmid comprising recombinant DNA as claimed in any one of claims 1 to 12.

14. A plasmid as claimed in claim 13 comprising a plasmid suitable for transformation of E.coli or yeast into which the recombinant DNA has been inserted.

15. A plasmid as claimed in claim 13 or claim 14 comprising pBR322, pACYC184 or pUC18 into which the recombinant DNA has been inserted.

16. A method for producing a plasmid as claimed in claim 15 comprising the following steps:
(a) extracting total genomic DNA from an S. enteritidis or a SEFA expressing S. dublin to produce the recombinant DNA;
(b) partially digesting the genomic DNA with SauIIIA restriction endonuclease to provide fragments in the size range 5 to 10 kilobases;
(c) ligating the fragments into a plasmid pBR322, pACYC184 or pUC18 and,
(d) selecting desired plasmids for their ability to express SEFA, or an epitopic part thereof being characterised in that it is capable of specific binding with monoclonal antibody secreted by at least one of the hybridoma cell lines deposited at the ECACC under the accession numbers 90101101 and 90121902.

17. A method as claimed in claim 16 wherein the desired plasmid comprises a fragment comprising Sequences I and II of claim 3 contiguously and the method further comprises the step of ligating a further DNA sequence into the BamH1 site between the Sequences and in frame with the Sequences.

18. A plasmid obtainable by the method of claim 16 or claim 17.

19. A transformant microorganism comprising a plasmid as claimed in any one of claims 13, 14, 15 or 18.

20. A microorganism as claimed in claim 19 wherein the plasmid host is a yeast or an E.coli.

21. A microorganism as claimed in claim 20 wherein the plasmid host is an E. coli DH5alpha.

22. A polypeptide encoded by the recombinant DNA of claim 11.

23. A test kit for the identification of microorganisms as being of either serotype S. enteritidis or S. dublin comprising a polypeptide or oligopeptide comprising SEFA or an epitopic part thereof as expressed by a transformant as claimed in any one of claims 20 to 22 the epitopic part being characterised in that it is capable of specific binding with monoclonal antibody secreted by at least one of the hybridoma cell lines deposited at the ECACC under the accession numbers 90101101 and 90121902.

24. A method for the determination of the presence of microorganisms having DNA or RNA polynucleotide sequence encoding SEFA or such DNA or RNA itself, comprising:
(a) providing a sample suspected of containing said encoding polynucleotide sequence;
(b) determining the presence of said sequence by monitoring hybridization of SEFA sequence targeted polynucleotide hybridization probes with said DNA or RNA.

25. A method as claimed in claim 24 wherein the polynucleotide probes are targeted to any one of contiguous Sequence pairs I and II; III and IV; or V and VI of claims 3 to 5.

26. A method as claimed in claim 25 wherein the polynucleotide probe consists of contiguous Sequence pairs I and II; III and IV; or V and VI of claims 3 to 5.

27. A test kit for performing the method of any one of claims 24 to 26 comprising polynucleotide hybridization probes targeted at the contiguous Sequence pairs III and IV or V and VI of claim 4 and claim 5.

28. A test kit as claimed in claim 27 wherein the probes comprise sequences comprising contiguous Sequence pairs III and IV or V and VI of claim 4 and claim 5.

29. A method for determining the presence of microorganisms having DNA or RNA polynucleotide sequence encoding for SEFA or such DNA or RNA itself, comprising:
(a) providing a sample suspected of containing said encoding polynucleotide sequence;
(b) subjecting said sample to conditions under which polynucleotide sequences comprising Sequences I and II of claim 2 are replicated by use of the polymerase chain reaction;
(c) determining the presence of any sequence produced.

30. A method as claimed in claim 29 wherein the step (c) is carried out using a polynucleotide hybridization probe.

31. A method as claimed in claim 29 or claim 30 wherein step (b) employs primer pairs comprising one primer selected from group (A) and the other from group (B):

Group A:                                    Group B:

5' -GTGCGAATGCTAATAGTTGA- 3'     5' -AAAACAGGCTGTCCTTGTCCA- 3'

5' -TGCGTAAATCAGCATCTGCA- 3'     5' -TTAGCGTTTCTTGAGAGCTGG- 3'

5' -TCTGCAGTAGCAGTTCTTGC- 3'     5' -TTTTGATACTGCTGAACGTAG- 3'

5' -GCTCAGAATACAACATCAGCCAA- 3'

32. A method as claimed in any one of claims 29 to 31 wherein the step (c) is carried out using an oligonucleotide probe selected from sequences of either of groups A or B (as described herein) which is different to that of either of the primers used for step (b).

33. A test kit for performing the method of any one of claims 29 to 32 comprising primers and probes having sequences selected from the groups (A) and (B).

## Patentansprüche

1. Rekombinierte DNA, welche
(a) die Aminosäuresequenz des Salmonellaenteritidis-Fimbrien-Antigens (SEFA): 
M L I V D F W R F C N M R K S A S A V A V L A L I A C G S A H A A G F
V G N K A E V Q A A V T I A A Q N T T S A N W S Q D P G F T G P A V A
A G Q K V G T L S I T A T G P H N S V S I A G K G A S V S G G V A T V
P F V D G Q G Q P V F R G R I Q G A N I N D Q A N T G I D G L A G W R
V A S S Q E T L N V P V T T F G K S T L P A G T F T A T F Y V Q Q Y Q
N
(b) einen epitopen Teil davon, oder
(c) eine allele Variante von beiden kodiert,
wobei der epitope Teil und die allele Variante dadurch gekennzeichnet sind, daß sie spezifisch mit einem monoklonalen Antikörper binden, der von mindestens einer der bei der ECACC mit den Zugangs-Nrn. 90101101 und 90121902 hinterlegten Hybridomzellinien sezerniert wird.

2. Rekombinierte DNA nach Anspruch 1, wobei geeignete flankierende Sequenzen zur Kontrolle der Aminosäureexpression vorgesehen sind.

3. Rekombinierte DNA nach Anspruch 1 oder 2, welche die Sequenzen I und II umfaßt: 
Sequenz I

5'- G CTCAGAATAC AACATCAGCC AACTGGAGTC AGGAT -3'
3'- C GAGTCTTATG TTGTAGTCGG TTGACCTCAG TCCTA -5'
230 240 250
 
Sequenz II

5'- CCTGG CTTTACAGGG CCTGCTGTTG CTGCTGGTCA GAAAGTTGGT
3'- GGACC GAAATGTCCC GGACGACAAC GACGACCAGT CTTTCAACCA
260 270 280 290 300

ACTCTCAGCA TTACTGCTAC TGGTCCACAT AACTCAGTAT CTATTGCAGG TAAAGGGGCT
TGAGAGTCGT AATGACGATG ACCAGGTGTA TTGAGTCATA GATAACGTCC ATTTCCCCGA
310 320 330 340 350 360

TCGGTATCTG GTGGTGTAGC CACTGTCCCG TTCGTTGATG GACAAGGACA GCCTGTTTT -3'
AGCCATAGAC CACCACATCG GTGACAGGGC AAGCAACTAC CTGTTCCTGT CGGACAAAA -5'
370 380 390 400 410
 oder Sequenzen, die dazu äquivalent degeneriert sind.

4. Rekombinierte DNA nach einem der vorhergehenden Ansprüche, welche die Sequenzen III und IV umfaßt: 
Sequenz III

5'- ATGCTAAT AGTTGATTTT TGGAGATTTT GTAATATGCG TAAATCAGCA
3'- TACGATTA TCAACTAAAA ACCTCTAAAA CATTATACGC ATTTAGTCGT
80 90 100 110 120

TCTGCAGTAG CAGTTCTTGC TTTAATTGCA TGTGGCAGTG CCCACGCAGC TGGCTTTGTT
AGACGTCATC GTCAAGAACG AAATTAACGT ACACCGTCAC GGGTGCGTCG ACCGAAACAA
130 140 150 160 170 180

GGTAACAAAG CAGAGGTTCA GGCAGCGGTT ACTATTGCAG CTCAGAATAC AACATCAGCC
CCATTGTTTC GTCTCCAAGT CCGTCGCCAA TGATAACGTC GAGTCTTATG TTGTAGTCGG
190 200 210 220 230 240

AACTGGAGTC AGGAT -3'
TTGACCTCAG TCCTA -5'
250
 
Sequenz IV

5'- CCTGG CTTTACAGGG CCTGCTGTTG CTGCTGGTCA GAAAGTTGGT
3'- GGACC GAAATGTCCC GGACGACAAC GACGACCAGT CTTTCAACCA
260 270 280 290 300

ACTCTCAGCA TTACTGCTAC TGGTCCACAT AACTCAGTAT CTATTGCAGG TAAAGGGGCT
TGAGAGTCGT AATGACGATG ACCAGGTGTA TTGAGTCATA GATAACGTCC ATTTCCCCGA
310 320 330 340 350 360

TCGGTATCTG GTGGTGTAGC CACTGTCCCG TTCGTTGATG GACAAGGACA GCCTGTTTTC
AGCCATAGAC CACCACATCG GTGACAGGGC AAGCAACTAC CTGTTCCTGT CGGACAAAAG
370 380 390 400 410 420
 
CGTGGGCGTA TTCAGGGAGC CAATATTAAT GACCAAGCAA ATACTGGAAT TGACGGGCTT
GCACCCGCAT AAGTCCCTCG GTTATAATTA CTGGTTCGTT TATGACCTTA ACTGCCCGAA
430 440 450 460 470 480

GCAGGTTGGC GAGTTGCCAG CTCTCAAGAA ACGCTAAATG TCCCTGTCAC AACCTTTGGT
CGTCCAACCG CTCAACGGTC GAGAGTTCTT TGCGATTTAC AGGGACAGTG TTGGAAACCA
490 500 510 520 530 540

AAATCGACCC TGCCAGCAGG TACTTTCACT GCGACCTTCT ACGTTCAGCA GTATCAAAAC -3'
TTTAGCTGGG ACGGTCGTCC ATGAAAGTGA CGCTGGAAGA TGCAAGTCGT CATAGTTTTG -5'
550 560 570 580 590 600
 oder Sequenzen, die dazu äquivalent degeneriert sind.

5. Rekombinierte DNA nach einem der vorhergehenden Ansprüche, welche die Sequenzen V und VI umfaßt: 
Sequenz V

5'- GATCCTTGTT TTTTTTCTTA AATTTTTAAA ATGGCGTGAG TATATTAGCA TCCGCACAGA
3'- CTAGGAACAA AAAAAAGAAT TTAAAAATTT TACCGCACTC ATATAATCGT AGGCGTGTCT
10 20 30 40 50 60

TAAATTGTGC GAATGCTAAT AGTTGATTTT TGGAGATTTT GTAATATGCG TAAATCAGCA
ATTTAACACG CTTACGATTA TCAACTAAAA ACCTCTAAAA CATTATACGC ATTTAGTCGT
70 80 90 100 110 120
 
TCTGCAGTAG CAGTTCTTGC TTTAATTGCA TGTGGCAGTG CCCACGCAGC TGGCTTTGTT
AGACGTCATC GTCAAGAACG AAATTAACGT ACACCGTCAC GGGTGCGTCG ACCGAAACAA
130 140 150 160 170 180

GGTAACAAAG CAGAGGTTCA GGCAGCGGTT ACTATTGCAG CTCAGAATAC AACATCAGCC
CCATTGTTTC GTCTCCAAGT CCGTCGCCAA TGATAACGTC GAGTCTTATG TTGTAGTCGG
190 200 210 220 230 240

AACTGGAGTC AGGAT -3'
TTGACCTCAG TCCTA -5'
250
 
Sequenz VI

5'- CCTGG CTTTACAGGG CCTGCTGTTG CTGCTGGTCA GAAAGTTGGT
3'- GGACC GAAATGTCCC GGACGACAAC GACGACCAGT CTTTCAACCA
260 270 280 290 300
 
ACTCTCAGCA TTACTGCTAC TGGTCCACAT AACTCAGTAT CTATTGCAGG TAAAGGGGCT
TGAGAGTCGT AATGACGATG ACCAGGTGTA TTGAGTCATA GATAACGTCC ATTTCCCCGA
310 320 330 340 350 360

TCGGTATCTG GTGGTGTAGC CACTGTCCCG TTCGTTGATG GACAAGGACA GCCTGTTTTC
AGCCATAGAC CACCACATCG GTGACAGGGC AAGCAACTAC CTGTTCCTGT CGGACAAAAG
370 380 390 400 410 420

CGTGGGCGTA TTCAGGGAGC CAATATTAAT GACCAAGCAA ATACTGGAAT TGACGGGCTT
GCACCCGCAT AAGTCCCTCG GTTATAATTA CTGGTTCGTT TATGACCTTA ACTGCCCGAA
430 440 450 460 470 480

GCAGGTTGGC GAGTTGCCAG CTCTCAAGAA ACGCTAAATG TCCCTGTCAC AACCTTTGGT
CGTCCAACCG CTCAACGGTC GAGAGTTCTT TGCGATTTAC AGGGACAGTG TTGGAAACCA
490 500 510 520 530 540

AAATCGACCC TGCCAGCAGG TACTTTCACT GCGACCTTCT ACGTTCAGCA GTATCAAAAC
TTTAGCTGGG ACGGTCGTCC ATGAAAGTGA CGCTGGAAGA TGCAAGTCGT CATAGTTTTG
550 560 570 580 590 600

TAATTTAATT TAAACTTTAT AAATGCCCTC AATATGAGCG AGTTTGGATA ATTTTATTAT
ATTAAATTAA ATTTGAAATA TTTACGGGAG TTATACTCGC TCAAACCTAT TAAAATAATA
610 620 630 640 650 660

TTTAAAAATA TCTATTTTGA ATAGATAGGT TTTATGCTTC CATGCAAAAA CTTAAAGAGG
AAATTTTTAT AGATAAAACT TATCTATCCA AAATACGAAG GTACGTTTTT GAATTTCTCC
670 680 690 700 710 720
 
GATTATGTAT ATTTTGAATA AATTTATACG TAGAACTGTT ATCTTTTTCC TTTTTTTTGC
CTAATACATA TAAAACTTAT TTAAATATGC ATCTTGACAA TAGAAAAAGG AAAAAAAACG
730 740 750 760 770 780

TACCTTCCAA TTGCTTCTTC GGAAAGTAAA AAAATTGAGC AACCATTATT AACACAAAAA
ATGGAAGGTT AACGAAGAAG CCTTTCATTT TTTTAACTCG TTGGTAATAA TTGTGTTTTT
790 800 810 820 830 840

TATTATGGCC TAAGATTGGG CACTACACGT GTTATTTATA AAGAAGATGC TCCATCAACA
ATAATACCGG ATTCTAACCC GTGATGTGCA CAATAAATAT TTCTTCTACG AGGTAGTTGT
850 860 870 880 890 900

AGTTTTTGGA TTATGAATGA AAAAGAATAT CCAATCCTTG TTCAAACTCA AGTATATAAT
TCAAAAACCT AATACTTACT TTTTCTTATA GGTTAGGAAC AAGTTTGAGT TCATATATTA
910 920 930 940 950 960

GATGATAAAT CATCAAAAGC TCCATTTATT GTAACACCAC CTATTTTGAA AGTTGAAAGT
CTACTATTTA GTAGTTTTCG AGGTAAATAA CATTGTGGTG GATAAAACTT TCAACTTTCA
970 980 990 1000 1010 1020

AATGCGCGAA CAAGATTGAA GGTAATACCA ACAAGTAATC TATTCAATAA AAATGAGGAG
TTACGCGCTT GTTCTAACTT CCATTATGGT TGTTCATTAG ATAAGTTATT TTTACTCCTC
1030 1040 1050 1060 1070 1080

TCTTTGTATT GGTTGTGTGT AAAAGGAGTC CCACCACTAA ATGATAATGA AAGCAATAAT
AGAAACATAA CCAACACACA TTTTCCTCAG GGTGGTGATT TACTATTACT TTCGTTATTA
1090 1100 1110 1120 1130 1140
 
AAAAACAACA TAACTACGAA TCTTAATGTG AATGTGGTTA CGAATAGTTG TATTAAATTA
TTTTTGTTGT ATTGATGCTT AGAATTACAC TTACACCAAT GCTTATCAAC ATAATTTAAT
1150 1160 1170 1180 1190 1200

ATTTATAGGC CTAAAACTAT AGACTTAACG ACAATGGAGA TTGCAGATAA ATTAAAGTTA
TAAATATCCG GATTTTGATA TCTGAATTGC TGTTACCTCT AACGTCTATT TAATTTCAAT
1210 1220 1220 1240 1250 1260

GAGAGAAAAG GAAATAGTAT AGTTATAAAG AATCCAACAT CATCATATGT GAATATTGCA
CTCTCTTTTC CTTTATCATA TCAATATTTC TTAGGTTGTA GTAGTATACA CTTATAACGT
1270 1280 1290 1300 1310 1320

AATATTAAAT CTGGTAATTT AAGTTTTAAT ATTCCAAATG GATATATTGA GCCATTTGGA
TTATAATTTA GACCATTAAA TTCAAAATTA TAAGGTTTAC CTATATAACT CGGTAAACCT
1330 1340 1350 1360 1370 1380

TATGCTCAAT TACCTGGTGG AGTACATAGT AAAATAACTT TGACTATTTT GGATGATAAC
ATACGAGTTA ATGGACCACC TCATGTATCA TTTTATTGAA ACTGATAAAA CCTACTATTG
1390 1400 1410 1020 1430 1440

GGCGCTGAAA TTATAAGAGA ATTATTAGTT TAAGGTGTAA AACAAATGAA GAAAACCACA
CCGCGACTTT AATATTCTCT TAATAATCAA ATTCCACATT TTGTTTACTT CTTTTGGTGT
1450 1460 1470 1480 1490 1500

ATTACTCTAT TTGTTTTAAC CAGTGTATTT CACTCTGGAA ATGTTTTCTC CAGACAATAT
TAATGAGATA AACAAAATTG GTCACATAAA GTGAGACCTT TACAAAAGAG GTCTGTTATA
1510 1520 1530 1540 1550 1560
 
AATTTCGACT ATGGAAGTTT GAGTCTTCTC CCGGTGAGAA TGCATCTTTT CTAAGTGTTG
TTAAAGCTGA TACCTTCAAA CTCAGAAGAG GGCCACTCTT ACGTAGAAAA GATTCACAAC
1570 1580 1590 1600 1610 1620

AAACGCTTCC CTGGTAATTA TGTTGTTGAT GTATATTTGA ATAATCAGTT AAAAGAAACT
TTTGCGAAGG GACCATTAAT ACAACAACTA CATATAAACT TATTAGTCAA TTTTCTTTGA
1630 1640 1650 1660 1670 1680

ACTGAGTTGT ATTTCAAATC AATGACTCAG ACTCTAGAAC CATGCTTAAC AAAAGAAAAA
TGACTCAACA TAAAGTTTAG TTACTGAGTC TGAGATCTTG GTACGAATTG TTTTCTTTTT
1690 1700 1710 1720 1730 1740

CTTATAAAGT ATGGGATCGC CATCCAGGAG CTTCATGGGT TGCAGTTTGA TAATGAACAA
GAATATTTCA TACCCTAGCG GTAGGTCCTC GAAGTACCCA ACGTCAAACT ATTACTTGTT
1750 1760 1770 1780 1790 1800

TGCGTTCTCT TAGAGCATTC TCCTCTTTAA ATATACTTAT AACGCGGCTA ACCAAAGTTT
ACGCAAGAGA ATCTCGTAAG AGGAGAAATT TATATGAATA TTGCGCCGAT TGGTTTCAAA
1810 1820 1830 1840 1850 1860

GCTTTTAAAT GCACCATCTA AAATTCTATC TCCAATAGAC AGTGAAATTG CTGATGAAAA
CGAAAATTTA CGTGGTAGAT TTTAAGATAG AGGTTATCTG TCACTTTAAC GACTACTTTT
1870 1880 1890 1900 1910 1920

TATCTGGGAT GATGGCATTA ACGCTTTTCT TTTAAATTAC AGAGCTTAAT TATTTGCATT
ATAGACCCTA CTACCGTAAT TGCGAAAAGA AAATTTAATG TCTCGAATTA ATAAACGTAA
1930 1940 1950 1960 1970 1980
 
CTAAGGTTGG AGGAGAGAGA TTCATACTTT GGTCAAATTC AACCTTGGTT TTAATTTTGG
GATTCCAACC TCCTCTCTCT AAGTATGAAA CCAGTTTAAG TTGGAACCAA AATTAAAACC
1990 2000 2010 2020 2030 2040

TCCCTGGCGG CTAAGGAATC TATCATCTTG GCAAAACTTG TCAAGCGAAA AAAAATTTGA
AGGGACCGCC GATTCCTTAG ATAGTAGAAC AGTTTTGAAC AGTTCGCTTT TTTTTAAACT
2050 2060 2070 2080 2090 2100

ATCAGCATAT ATTTATGCTG AGCGAGGTTT AAAAAAAATA AAGAGCAAAC TAACAGTTGG
TAGTCGTATA TAAATACGAC TCGCTCCAAA TTTTTTTTAT TTCTCGTTTG ATTGTCAACC
2110 2120 2130 2140 2150 2160

GGACAAATAT ACCAGTGCAG ATTTATTCGA TAGCGTACCA TTTAGAGGCT TTTCTTTAAA
CCTGTTTATA TGGTCACGTC TAAATAAGCT ATCGCATGGT AAATCTCCGA AAAGAAATTT
2170 2180 2190 2200 2210 2220

TAAAGATGAA AGTATGATAC CTTTCTCACA GAGAACATAT TATCCAACAA TACGTGGTAT
ATTTCTACTT TCATACTATG GAAAGAGTGT CTCTTGTATA ATAGGTTGTT ATGCACCATA
2230 2240 2250 2260 2270 2280

TGCGAAAACC AATGCGACTG TAGAAGTAAG ACAAAATGGA TACTTGATAT ATTCTACTTC
ACGCTTTTGG TTACGCTGAC ATCTTCATTC TGTTTTACCT ATGAACTATA TAAGATGAAG
2290 2300 2310 2320 2330 2340

AGTCCCCCCC GGGCAATTCG AGATAGGTAG AGAACAAATT GCTGATC -3'
TCAGGGGGGG CCCGTTAAGC TCTATCCATC TCTTGTTTAA CGACTAG -5'
2350 2360 2370 2380
 oder Sequenzen, die dazu äquivalent degeneriert sind.

6. Rekombinierte DNA nach Anspruch 3, wobei die Sequenzen I und II in einer aneinandergrenzenden Sequenz enthalten sind.

7. Rekombinierte DNA nach Anspruch 4, wobei die Sequenzen III und IV in einer aneinandergrenzenden Sequenz enthalten sind.

8. Rekombinierte DNA nach Anspruch 5, wobei die Sequenzen V und VI in einer aneinandergrenzenden Sequenz enthalten sind.

9. Rekombinierte DNA nach einem der Ansprüche 1 bis 5, die ferner eine Sequenz aufweist, die eine weitere Aminosäuresequenz kodiert.

10. Rekombinierte DNA nach Anspruch 9, wobei die weitere Aminosäuresequenz zusätzlich epitope Teile von SEFA aufweist, wobei die epitopen Teile dadurch gekennzeichnet sind, daß sie spezifisch mit monoklonalen Antikörper binden können, die von mindestens einer der bei der ECACC mit den Zugangs-Nrn. 90101101 und 90121902 hinterlegten Hybridomzellninien sezerniert werden.

11. Rekombinierte DNA nach Anspruch 9, wobei die weitere Aminosäuresequenz eine nicht-SEFA-epitope Sequenz umfaßt.

12. Rekombinierte DNA nach Anspruch 11, wobei die nicht-SEFA-epitope Sequenz das SB10-Epitop von Mycobacterium bovis umfaßt.

13. Neues Plasmid, welches rekombinierte DNA nach einem der Ansprüche 1 bis 12 enthält.

14. Plasmid nach Anspruch 13, das ein Plasmid umfaßt, das zur Transformation von E. coli oder Hefe geeignet ist, in das rekombinierte DNA eingeführt worden ist.

15. Plasmid nach Anspruch 13 oder 14, das pBR322, pACYC184 oder pUC18, in die rekombinierte DNA eingeführt worden ist, umfaßt.

16. Verfahren zur Herstellung eines Plasmids nach Anspruch 15, bei dem:
(a) die gesamte genomische DNA aus S. enteritidis oder SEFA exprimierendem S. dublin zur Herstellung der rekombinierten DNA extrahiert wird,
(b) die genomischen DNA mit der Restriktionsendonuklease SauIIIA zur Bereitstellung von Fragmenten im Größenbereich von 5 bis 10 Kilobasen zum Teil verdaut wird,
(c) die Fragmente in das Plasmid pBR322, pACYC184 oder pUC18 ligiert werden und
(d) gewünschte Plasmide selektiert werden, die zur Expression von SEFA oder epitope Teile davon, die dadurch gekennzeichnet sind, daß sie spezifisch mit einem monoklonalen Antikörper binden können, der von mindestens einer der bei der ECACC mit den Zugangs-Nrn. 90101101 und 90121902 hinterlegten Hybridomzellen sezerniert wird, befähigt sind.

17. Verfahren nach Anspruch 16, wobei das erwünschte Plasmid ein Fragment umfaßt, das die Sequenzen I und II von Anspruch 3 aneinandergrenzend enthält, wobei das Verfahren ferner aufweist, daß eine weitere DNA-Sequenz in die BamH1-Stelle zwischen den Sequenzen und im Leseraster mit den Sequenzen ligiert wird.

18. Plasmid, das nach dem Verfahren von Anspruch 16 oder Anspruch 17 erhältlich ist.

19. Transformierter Mikroorganismus, welcher ein Plasmid nach einem der Ansprüche 13, 14, 15 oder 18 enthält.

20. Mikroorganismus nach Anspruch 19, wobei der Plasmidwirt eine Hefe oder ein E. coli ist.

21. Mikroorganism nach Anspruch 20, wobei der Plasmidwirt ein E. coli DH5alpha ist.

22. Polypeptid, das durch die rekombinierte DNA von Anspruch 11 kodiert wird.

23. Testkit zur Identifizierung von Mikroorganismen als entweder Serotyp S. enteritidis oder S. dublin, das ein Polypeptid oder Oligopeptid aufweist, welche SEFA oder einen epitopen Teil davon umfaßt, das von einer Transformante nach einem der Ansprüche 20 bis 22 exprimiert wird, wobei der epitope Teil dadurch gekennzeichnet ist, daß er spezifisch mit einem monoklonalen Antikörper binden kann, der von mindestens einer der bei der ECACC mit den Zugangs-Nrn. 90101101 und 90121902 hinterlegten Hybridomzellinien sezerniert wird.

24. Verfahren zur Bestimmung des Vorliegens von Mikroorganismen mit SEFA kodierender DNA- oder RNA-Polynukleotidsequenz, oder derartiger DNA oder RNA als solcher, bei dem:
(a) eine Probe bereitgestellt wird, die vermutlich die kodierende Polynukleotidsequenz enthält,
(b) das Vorliegen der Sequenz bestimmt wird, indem die Hybridisierung von Polynukleotid-Hybridisierungssonden, die auf die SEFA-Sequenz als Ziel gerichtet sind, mit der DNA oder RNA verfolgt wird.

25. Verfahren nach Anspruch 24, wobei die Polynukleotidsonden auf eine der aneinandergrenzenden Sequenzpaare I und II, III und IV oder V und VI der Ansprüche 3 bis 5 als Ziel gerichtet sind.

26. Verfahren nach Anspruch 25, wobei die Polynukleotidsonde aus den aneinandergrenzenden Sequenzpaaren I und II, III und IV oder V und VI der Ansprüche 3 bis 5 besteht.

27. Testkit zur Durchführung des Verfahrens nach einem der Ansprüche 24 bis 26, das Polynukleotid-Hybridisierungssonden aufweist, die auf die aneinandergrenzenden Sequenzpaare III und IV oder V und VI von Anspruch 4 und Anspruch 5 als Ziel gerichtet sind.

28. Testkit nach Anspruch 27, wobei die Sonden Sequenzen aufweisen, welche die aneinandergrenzenden Sequenzpaare III und IV oder V und VI von Anspruch 4 und Anspruch 5 umfassen.

29. Verfahren zur Bestimmung des Vorliegens von Mikroorganismen mit SEFA kodierender DNA- oder RNA-Polynukleotidsequenz, oder derartiger DNA oder RNA als solcher, bei dem:
(a) eine Probe bereitgestellt wird, die vermutlich die kodierende Polynukleotidsequenz enthält,
(b) die Probe Bedingungen ausgesetzt wird, unter denen Polynukleotidsequenzen, welche die Sequenzen I und II von Anspruch 2 aufweisen, durch Anwendung der Polymerase-Kettenreaktion repliziert werden,
(c) das Vorliegen einer hergestellten Sequenz bestimmt wird.

30. Verfahren nach Anspruch 29, wobei der Schritt (c) unter Verwendung einer Polynukleotid-Hybridisierungssonde durchgeführt wird.

31. Verfahren nach Anspruch 29 oder 30, wobei in Schritt (b) Primerpaare verwendet werden, wobei ein Primer aus Gruppe (A) und der andere aus Gruppe (B) ausgewählt ist:

Gruppe A: | Gruppe B:

5' -GTGCGAATGCTAATAGTTGA- 3' | 5' -AAAACAGGCTGTCCTTGTCCA- 3'

5' -TGCGTAAATCAGCATCTGCA- 3' | 5' -TTAGCGTTTCTTGAGAGCTGG- 3'

5' -TCTGCAGTAGCAGTTCTTGC- 3' | 5' -TTTTGATACTGCTGAACGTAG- 3'

5' -GCTCAGAATACAACATCAGCCAA- 3'

32. Verfahren nach einem der Ansprüche 29 bis 31, wobei Schritt (c) unter Verwendung einer Oligonukleotidsonde durchgeführt wird, die unter den Sequenzen jeder der Gruppen A oder B (wie oben beschrieben) ausgewählt ist, welche sich von der unterscheidet, die jeder der in Schritt (b) verwendeten Primer hat.

33. Testkit zur Durchführung des Verfahrens nach einem der Ansprüche 29 bis 32, der Primer und Sonden aufweist, die Sequenzen haben, welche aus den Gruppen (A) und (B) ausgewählt sind.

## Revendications

1. ADN recombinant, codant
(a) la séquence d'aminoacides de l'antigène de fimbriae de Salmonella enteritidis (SEFA) 
M L I V D F W R F C N M R K S A S A V A V L A L I A C G S A H A A G F
V G N K A E V Q A A V T I A A Q N T T S A N W S Q D P G F T G P A V A
A G Q K V G T L S I T A T G P H N S V S I A G K G A S V S G G V A T V
P F V D G Q G Q P V F R G R I Q G A N I N D Q A N T G I D G L A G W R
V A S S Q E T L N V P V T T F G K S T L P A G T F T A T F Y V Q Q Y Q
N
(b) une partie épitopique de cette séquence ou
(c) un variant allélique de l'une ou l'autre,
la partie épitopique et le variant allélique étant caractérisés en ce qu'ils sont capables de liaison spécifique avec un anticorps monoclonal sécrété par au moins l'une des lignées cellulaires d'hybridome déposées auprès de l'ECACC sous les numéros de dépôt 90101101 et 90121902.

2. ADN recombinant suivant la revendication 1, dans lequel des séquences adjacentes convenables sont prévues pour contrôler l'expression de la séquence d'aminoacides.

3. ADN recombinant suivant la revendication 1 ou la revendication 2, comprenant les séquences I et II : 
Séquence I

5'- G CTCAGAATAC AACATCAGCC AACTGGAGTC AGGAT -3'
3'- C GAGTCTTATG TTGTAGTCGG TTGACCTCAG TCCTA -5'
230 240 250
 
5'- CCTGG CTTTACAGGG CCTGCTGTTG CTGCTGGTCA GAAAGTTGGT
3'- GGACC GAAATGTCCC GGACGACAAC GACGACCAGT CTTTCAACCA
260 270 280 290 300

ACTCTCAGCA TTACTGCTAC TGGTCCACAT AACTCAGTAT CTATTGCAGG TAAAGGGGCT
TGAGAGTCGT AATGACGATG ACCAGGTGTA TTGAGTCATA GATAACGTCC ATTTCCCCGA
310 320 330 340 350 360

TCGGTATCTG GTGGTGTAGC CACTGTCCCG TTCGTTGATG GACAAGGACA GCCTGTTTT -3'
AGCCATAGAC CACCACATCG GTGACAGGGC AAGCAACTAC CTGTTCCTGT CGGACAAAA -5'
370 380 390 400 410
 ou des séquences qui en sont l'équivalent par dégénérescence.

4. ADN recombinant suivant l'une quelconque des revendications précédentes, comprenant les séquences III et IV : 
Séquence III

5'- ATGCTAAT AGTTGATTTT TGGAGATTTT GTAATATGCG TAAATCAGCA
3'- TACGATTA TCAACTAAAA ACCTCTAAAA CATTATACGC ATTTAGTCGT
80 90 100 110 120

TCTGCAGTAG CAGTTCTTGC TTTAATTGCA TGTGGCAGTG CCCACGCAGC TGGCTTTGTT
AGACGTCATC GTCAAGAACG AAATTAACGT ACACCGTCAC GGGTGCGTCG ACCGAAACAA
130 140 150 160 170 180

GGTAACAAAG CAGAGGTTCA GGCAGCGGTT ACTATTGCAG CTCAGAATAC AACATCAGCC
CCATTGTTTC GTCTCCAAGT CCGTCGCCAA TGATAACGTC GAGTCTTATG TTGTAGTCGG
190 200 210 220 230 240
 
AACTGGAGTC AGGAT -3'
TTGACCTCAG TCCTA -5'
250
 
Séquence IV

5'- CCTGG CTTTACAGGG CCTGCTGTTG CTGCTGGTCA GAAAGTTGGT
3'- GGACC GAAATGTCCC GGACGACAAC GACGACCAGT CTTTCAACCA
260 270 280 290 300

ACTCTCAGCA TTACTGCTAC TGGTCCACAT AACTCAGTAT CTATTGCAGG TAAAGGGGCT
TGAGAGTCGT AATGACGATG ACCAGGTGTA TTGAGTCATA GATAACGTCC ATTTCCCCGA
310 320 330 340 350 360

TCGGTATCTG GTGGTGTAGC CACTGTCCCG TTCGTTGATG GACAAGGACA GCCTGTTTTC
AGCCATAGAC CACCACATCG GTGACAGGGC AAGCAACTAC CTGTTCCTGT CGGACAAAAG
370 380 390 400 410 420

CGTGGGCGTA TTCAGGGAGC CAATATTAAT GACCAAGCAA ATACTGGAAT TGACGGGCTT
GCACCCGCAT AAGTCCCTCG GTTATAATTA CTGGTTCGTT TATGACCTTA ACTGCCCGAA
430 440 450 460 470 480

GCAGGTTGGC GAGTTGCCAG CTCTCAAGAA ACGCTAAATG TCCCTGTCAC AACCTTTGGT
CGTCCAACCG CTCAACGGTC GAGAGTTCTT TGCGATTTAC AGGGACAGTG TTGGAAACCA
490 500 510 520 530 540

AAATCGACCC TGCCAGCAGG TACTTTCACT GCGACCTTCT ACGTTCAGCA GTATCAAAAC -3'
TTTAGCTGGG ACGGTCGTCC ATGAAAGTGA CGCTGGAAGA TGCAAGTCGT CATAGTTTTG -5'
550 560 570 580 590 600
 ou des séquences qui en sont l'équivalent par dégénérescence.

5. ADN recombinant suivant l'une quelconque des revendications précédentes, comprenant les séquences V et VI : 
Séquence V

5'- GATCCTTGTT TTTTTTCTTA AATTTTTAAA ATGGCGTGAG TATATTAGCA TCCGCACAGA
3'- CTAGGAACAA AAAAAAGAAT TTAAAAATTT TACCGCACTC ATATAATCGT AGGCGTGTCT
10 20 30 40 50 60

TAAATTGTGC GAATGCTAAT AGTTGATTTT TGGAGATTTT GTAATATGCG TAAATCAGCA
ATTTAACACG CTTACGATTA TCAACTAAAA ACCTCTAAAA CATTATACGC ATTTAGTCGT
70 80 90 100 110 120

TCTGCAGTAG CAGTTCTTGC TTTAATTGCA TGTGGCAGTG CCCACGCAGC TGGCTTTGTT
AGACGTCATC GTCAAGAACG AAATTAACGT ACACCGTCAC GGGTGCGTCG ACCGAAACAA
130 140 150 160 170 180

GGTAACAAAG CAGAGGTTCA GGCAGCGGTT ACTATTGCAG CTCAGAATAC AACATCAGCC
CCATTGTTTC GTCTCCAAGT CCGTCGCCAA TGATAACGTC GAGTCTTATG TTGTAGTCGG
190 200 210 220 230 240

AACTGGAGTC AGGAT -3'
TTGACCTCAG TCCTA -5'
250
 
Séquence VI

5'- CCTGG CTTTACAGGG CCTGCTGTTG CTGCTGGTCA GAAAGTTGGT
3'- GGACC GAAATGTCCC GGACGACAAC GACGACCAGT CTTTCAACCA
260 270 280 290 300
 
ACTCTCAGCA TTACTGCTAC TGGTCCACAT AACTCAGTAT CTATTGCAGG TAAAGGGGCT
TGAGAGTCGT AATGACGATG ACCAGGTGTA TTGAGTCATA GATAACGTCC ATTTCCCCGA
310 320 330 340 350 360

TCGGTATCTG GTGGTGTAGC CACTGTCCCG TTCGTTGATG GACAAGGACA GCCTGTTTTC
AGCCATAGAC CACCACATCG GTGACAGGGC AAGCAACTAC CTGTTCCTGT CGGACAAAAG
370 380 390 400 410 420

CGTGGGCGTA TTCAGGGAGC CAATATTAAT GACCAAGCAA ATACTGGAAT TGACGGGCTT
GCACCCGCAT AAGTCCCTCG GTTATAATTA CTGGTTCGTT TATGACCTTA ACTGCCCGAA
430 440 450 460 470 480

GCAGGTTGGC GAGTTGCCAG CTCTCAAGAA ACGCTAAATG TCCCTGTCAC AACCTTTGGT
CGTCCAACCG CTCAACGGTC GAGAGTTCTT TGCGATTTAC AGGGACAGTG TTGGAAACCA
490 500 510 520 530 540

AAATCGACCC TGCCAGCAGG TACTTTCACT GCGACCTTCT ACGTTCAGCA GTATCAAAAC
TTTAGCTGGG ACGGTCGTCC ATGAAAGTGA CGCTGGAAGA TGCAAGTCGT CATAGTTTTG
550 560 570 580 590 600

TAATTTAATT TAAACTTTAT AAATGCCCTC AATATGAGCG AGTTTGGATA ATTTTATTAT
ATTAAATTAA ATTTGAAATA TTTACGGGAG TTATACTCGC TCAAACCTAT TAAAATAATA
610 620 630 640 650 660

TTTAAAAATA TCTATTTTGA ATAGATAGGT TTTATGCTTC CATGCAAAAA CTTAAAGAGG
AAATTTTTAT AGATAAAACT TATCTATCCA AAATACGAAG GTACGTTTTT GAATTTCTCC
670 680 690 700 710 720
 
GATTATGTAT ATTTTGAATA AATTTATACG TAGAACTGTT ATCTTTTTCC TTTTTTTTGC
CTAATACATA TAAAACTTAT TTAAATATGC ATCTTGACAA TAGAAAAAGG AAAAAAAACG
730 740 750 760 770 780

TACCTTCCAA TTGCTTCTTC GGAAAGTAAA AAAATTGAGC AACCATTATT AACACAAAAA
ATGGAAGGTT AACGAAGAAG CCTTTCATTT TTTTAACTCG TTGGTAATAA TTGTGTTTTT
790 800 810 820 830 840

TATTATGGCC TAAGATTGGG CACTACACGT GTTATTTATA AAGAAGATGC TCCATCAACA
ATAATACCGG ATTCTAACCC GTGATGTGCA CAATAAATAT TTCTTCTACG AGGTAGTTGT
850 860 870 880 890 900

AGTTTTTGGA TTATGAATGA AAAAGAATAT CCAATCCTTG TTCAAACTCA AGTATATAAT
TCAAAAACCT AATACTTACT TTTTCTTATA GGTTAGGAAC AAGTTTGAGT TCATATATTA
910 920 930 940 950 960

GATGATAAAT CATCAAAAGC TCCATTTATT GTAACACCAC CTATTTTGAA AGTTGAAAGT
CTACTATTTA GTAGTTTTCG AGGTAAATAA CATTGTGGTG GATAAAACTT TCAACTTTCA
970 980 990 1000 1010 1020

AATGCGCGAA CAAGATTGAA GGTAATACCA ACAAGTAATC TATTCAATAA AAATGAGGAG
TTACGCGCTT GTTCTAACTT CCATTATGGT TGTTCATTAG ATAAGTTATT TTTACTCCTC
1030 1040 1050 1060 1070 1080

TCTTTGTATT GGTTGTGTGT AAAAGGAGTC CCACCACTAA ATGATAATGA AAGCAATAAT
AGAAACATAA CCAACACACA TTTTCCTCAG GGTGGTGATT TACTATTACT TTCGTTATTA
1090 1100 1110 1120 1130 1140
 
AAAAACAACA TAACTACGAA TCTTAATGTG AATGTGGTTA CGAATAGTTG TATTAAATTA
TTTTTGTTGT ATTGATGCTT AGAATTACAC TTACACCAAT GCTTATCAAC ATAATTTAAT
1150 1160 1170 1180 1190 1200

ATTTATAGGC CTAAAACTAT AGACTTAACG ACAATGGAGA TTGCAGATAA ATTAAAGTTA
TAAATATCCG GATTTTGATA TCTGAATTGC TGTTACCTCT AACGTCTATT TAATTTCAAT
1210 1220 1220 1240 1250 1260

GAGAGAAAAG GAAATAGTAT AGTTATAAAG AATCCAACAT CATCATATGT GAATATTGCA
CTCTCTTTTC CTTTATCATA TCAATATTTC TTAGGTTGTA GTAGTATACA CTTATAACGT
1270 1280 1290 1300 1310 1320

AATATTAAAT CTGGTAATTT AAGTTTTAAT ATTCCAAATG GATATATTGA GCCATTTGGA
TTATAATTTA GACCATTAAA TTCAAAATTA TAAGGTTTAC CTATATAACT CGGTAAACCT
1330 1340 1350 1360 1370 1380

TATGCTCAAT TACCTGGTGG AGTACATAGT AAAATAACTT TGACTATTTT GGATGATAAC
ATACGAGTTA ATGGACCACC TCATGTATCA TTTTATTGAA ACTGATAAAA CCTACTATTG
1390 1400 1410 1020 1430 1440

GGCGCTGAAA TTATAAGAGA ATTATTAGTT TAAGGTGTAA AACAAATGAA GAAAACCACA
CCGCGACTTT AATATTCTCT TAATAATCAA ATTCCACATT TTGTTTACTT CTTTTGGTGT
1450 1460 1470 1480 1490 1500

ATTACTCTAT TTGTTTTAAC CAGTGTATTT CACTCTGGAA ATGTTTTCTC CAGACAATAT
TAATGAGATA AACAAAATTG GTCACATAAA GTGAGACCTT TACAAAAGAG GTCTGTTATA
1510 1520 1530 1540 1550 1560
 
AATTTCGACT ATGGAAGTTT GAGTCTTCTC CCGGTGAGAA TGCATCTTTT CTAAGTGTTG
TTAAAGCTGA TACCTTCAAA CTCAGAAGAG GGCCACTCTT ACGTAGAAAA GATTCACAAC
1570 1580 1590 1600 1610 1620

AAACGCTTCC CTGGTAATTA TGTTGTTGAT GTATATTTGA ATAATCAGTT AAAAGAAACT
TTTGCGAAGG GACCATTAAT ACAACAACTA CATATAAACT TATTAGTCAA TTTTCTTTGA
1630 1640 1650 1660 1670 1680

ACTGAGTTGT ATTTCAAATC AATGACTCAG ACTCTAGAAC CATGCTTAAC AAAAGAAAAA
TGACTCAACA TAAAGTTTAG TTACTGAGTC TGAGATCTTG GTACGAATTG TTTTCTTTTT
1690 1700 1710 1720 1730 1740

CTTATAAAGT ATGGGATCGC CATCCAGGAG CTTCATGGGT TGCAGTTTGA TAATGAACAA
GAATATTTCA TACCCTAGCG GTAGGTCCTC GAAGTACCCA ACGTCAAACT ATTACTTGTT
1750 1760 1770 1780 1790 1800

TGCGTTCTCT TAGAGCATTC TCCTCTTTAA ATATACTTAT AACGCGGCTA ACCAAAGTTT
ACGCAAGAGA ATCTCGTAAG AGGAGAAATT TATATGAATA TTGCGCCGAT TGGTTTCAAA
1810 1820 1830 1840 1850 1860

GCTTTTAAAT GCACCATCTA AAATTCTATC TCCAATAGAC AGTGAAATTG CTGATGAAAA
CGAAAATTTA CGTGGTAGAT TTTAAGATAG AGGTTATCTG TCACTTTAAC GACTACTTTT
1870 1880 1890 1900 1910 1920

TATCTGGGAT GATGGCATTA ACGCTTTTCT TTTAAATTAC AGAGCTTAAT TATTTGCATT
ATAGACCCTA CTACCGTAAT TGCGAAAAGA AAATTTAATG TCTCGAATTA ATAAACGTAA
1930 1940 1950 1960 1970 1980
 
CTAAGGTTGG AGGAGAGAGA TTCATACTTT GGTCAAATTC AACCTTGGTT TTAATTTTGG
GATTCCAACC TCCTCTCTCT AAGTATGAAA CCAGTTTAAG TTGGAACCAA AATTAAAACC
1990 2000 2010 2020 2030 2040

TCCCTGGCGG CTAAGGAATC TATCATCTTG GCAAAACTTG TCAAGCGAAA AAAAATTTGA
AGGGACCGCC GATTCCTTAG ATAGTAGAAC AGTTTTGAAC AGTTCGCTTT TTTTTAAACT
2050 2060 2070 2080 2090 2100

ATCAGCATAT ATTTATGCTG AGCGAGGTTT AAAAAAAATA AAGAGCAAAC TAACAGTTGG
TAGTCGTATA TAAATACGAC TCGCTCCAAA TTTTTTTTAT TTCTCGTTTG ATTGTCAACC
2110 2120 2130 2140 2150 2160

GGACAAATAT ACCAGTGCAG ATTTATTCGA TAGCGTACCA TTTAGAGGCT TTTCTTTAAA
CCTGTTTATA TGGTCACGTC TAAATAAGCT ATCGCATGGT AAATCTCCGA AAAGAAATTT
2170 2180 2190 2200 2210 2220

TAAAGATGAA AGTATGATAC CTTTCTCACA GAGAACATAT TATCCAACAA TACGTGGTAT
ATTTCTACTT TCATACTATG GAAAGAGTGT CTCTTGTATA ATAGGTTGTT ATGCACCATA
2230 2240 2250 2260 2270 2280

TGCGAAAACC AATGCGACTG TAGAAGTAAG ACAAAATGGA TACTTGATAT ATTCTACTTC
ACGCTTTTGG TTACGCTGAC ATCTTCATTC TGTTTTACCT ATGAACTATA TAAGATGAAG
2290 2300 2310 2320 2330 2340

AGTCCCCCCC GGGCAATTCG AGATAGGTAG AGAACAAATT GCTGATC -3'
TCAGGGGGGG CCCGTTAAGC TCTATCCATC TCTTGTTTAA CGACTAG -5'
2350 2360 2370 2380
 ou des séquences qui en sont l'équivalent par dégénérescence.

6. ADN recombinant suivant la revendication 3, dans lequel les séquences I et II sont comprises dans une séquence contiguë.

7. ADN recombinant suivant la revendication 4, dans lequel les séquences III et IV sont comprises dans une séquence contiguë.

8. ADN recombinant suivant la revendication 3, dans lequel les séquences V et VI sont comprises dans une séquence contiguë.

9. ADN recombinant suivant l'une quelconque des revendications 1 à 5, comprenant en outre une séquence qui code une autre séquence d'aminoacides.

10. ADN recombinant suivant la revendication 9, dans lequel l'autre séquence d'aminoacides comprend d'autres parties épitopiques de l'antigène SEFA, ces parties épitopiques étant caractérisées en ce qu'elles sont capables de liaison spécifique avec l'anticorps monoclonal sécrété par l'une au moins des lignées cellulaires d'hybridome déposées auprès de l'ECACC sous les numéros de dépôt 90101101 et 92121902.

11. ADN recombinant suivant la revendication 9, dans lequel l'autre séquence d'aminoacides comprend une séquence non-épitopique de SEFA.

12. ADN recombinant suivant la revendication 11, dans lequel la séquence non-épitopique de SEFA comprend l'épitope SB10 de Mycobacterium bovis.

13. Plasmide nouveau comprenant l'ADN recombinant suivant l'une quelconque des revendications 1 à 12.

14. Plasmide suivant la revendication 13, comprenant un plasmide qui convient pour la transformation de E. coli ou d'une levure dans lequel l'ADN recombinant a été inséré.

15. Plasmide suivant la revendication 13 ou la revendication 14, comprenant le plasmide pBR322, pACYC184 ou pUC18 dans lequel l'ADN recombinant a été inséré.

16. Procédé de production d'un plasmide suivant la revendication 15, comprenant les étapes suivantes :
(a) extraction de l'ADN génomique total d'une S. enteritidis ou d'une S. dublin exprimant l'antigène SEFA pour produire l'ADN recombinant ;
(b) digestion partielle de l'ADN génomique avec l'endonucléase de restriction SauIIIA pour produire des fragments s'échelonnant dans la plage de 5 à 10 kilobases ;
(c) épissage des fragments en un plasmide pBR322, pACYC184 ou pUC18 et,
(d) sélection des plasmides désirés pour leur aptitude à exprimer l'antigène SEFA, ou une partie épitopique de cet antigène étant caractérisée par son aptitude à se lier spécifiquement à l'anticorps monoclonal sécrété par l'une au moins des lignées cellulaires d'hybridome déposée auprès de l'ECACC sous les numéros de dépôt 90101101 et 90121902.

17. Procédé suivant la revendication 16, dans lequel le plasmide désiré comprend un fragment constitué des séquences I et II suivant la revendication 3 en contiguïté et le procédé comporte en outre l'étape de ligation d'une autre séquence d'ADN dans le site de BamH1 entre les séquences et en phase avec les séquences.

18. Plasmide pouvant être obtenu par le procédé suivant la revendication 16 ou la revendication 17.

19. Micro-organisme transformant comprenant un plasmide suivant l'une quelconque des revendications 13, 14, 15 ou 18.

20. Micro-organisme suivant la revendication 19, dans lequel l'hôte du plasmide est une levure ou un E. coli.

21. Micro-organisme suivant la revendication 20, dans lequel l'hôte du plasmide est un E. coli DH5alpha.

22. Polypeptide codé par l'ADN recombinant de la revendication 11.

23. Kit analytique permettant d'identifier des micro-organismes comme appartenant au sérotype S. enteritidis ou S. dublin, comprenant un polypeptide ou un oligopeptide qui comprend l'antigène SEFA ou une partie épitopique de cet antigène comme exprimé par un transformant suivant l'une quelconque des revendications 20 à 22, la partie épitopique étant caractérisée en ce qu'elle est capable de liaison spécifique avec un anticorps monoclonal sécrété par l'une au moins des lignées cellulaires d'hybridome déposées auprès de l'EcACC sous les numéros de dépôt 90101101 et 90121902.

24. Méthode de détection de la présence de micro-organismes ayant une séquence polynucléotidique d'ADN ou d'ARN codant l'antigène SEFA ou cet ADN ou cet ARN lui-même, comprenant
(a) la prise d'un échantillon suspecté de contenir cette séquence polynucléotidique codante ;
(b) la détection de la présence de cette séquence par contrôle de l'hybridation avec cet ADN ou cet ARN de sondes d'hybridation polynucléotidiques ciblées sur la séquence de SEFA.

25. Méthode suivant la revendication 24, dans laquelle les sondes polynucléotidiques sont ciblées sur l'une quelconque des paires contiguës de séquences I et II, III et IV, ou V et VI suivant les revendications 3 à 5.

26. Méthode suivant la revendication 25, dans laquelle la sonde polynucléotidique consiste en paires de séquences contiguës I et II, III et IV, ou V et VI suivant les revendications 3 à 5.

27. Kit analytique pour l'application de la méthode suivant l'une quelconque des revendications 24 à 26, comprenant des sondes d'hybridation polynucléotidiques ciblées sur les paires de séquences contiguës III et IV, ou V et VI suivant la revendication 4 et la revendication 5.

28. Kit analytique suivant la revendication 27, dans lequel les sondes sont constituées de séquences comprenant des paires contiguës de Séquences III et IV, ou V et VI suivant la revendication 4 et la revendication 5.

29. Méthode de détection de la présence de micro-organismes ayant une séquence polynucléotidique d'ADN ou d'ARN codant l'antigène SEFA ou cet ADN ou cet ARN lui-même, comprenant :
(a) la prise d'un échantillon suspecté de contenir cette séquence polynucléotidique codante ;
(b) l'exposition de cet échantillon à des conditions dans lesquelles les séquences polynucléotidiques comprenant les séquences I et II suivant la revendication 2 sont répliquées par l'utilisation de la réaction de polymérisation en chaîne ;
(c) la détection de la présence de toute séquence produite.

30. Méthode suivant la revendication 29, dans laquelle l'étape (c) est conduite au moyen d'une sonde d'hybridation polynucléotidique.

31. Méthode suivant la revendication 29 ou la revendication 30, dans laquelle l'étape (b) emploie des paires d'amorces comprenant une amorce choisie dans le groupe (A) et une autre amorce choisie dans le groupe (B)

Groupe A

Groupe B

5' -GTGCGAATGCTAATAGTTGA- 3'

5' -AAAACAGGCTGTCCTTGTCCA- 3'

5' -TGCGTAAATCAGCATCTGCA- 3'

5' -TTAGCGTTTCTTGAGAGCTGG- 3'

5' -TCTGCAGTAGCAGTTCTTGC- 3'

5' -TTTTGATACTGCTGAACGTAG- 3'

5' -GCTCAGAATACAACATCAGCCAA- 3'

32. Méthode suivant l'une quelconque des revendications 29 à 31, dans laquelle l'étape (c) est conduite au moyen d'une sonde oligonucléotidique choisie dans les séquences du groupe (A) ou du groupe (B) (comme indiqué dans la description) qui est différente de celle de l'une ou l'autre des amorces utilisées dans l'étape (b).

33. Kit analytique pour l'application de la méthode suivant l'une quelconque des revendications 29 à 32, comprenant des amorces et des sondes ayant des séquences choisies dans les groupes (A) et (B).
